(19) **Europäisches Patentamt / European Patent Office / Office européen des brevets**

(11) **EP 3 212 211 B1**

(12) **EUROPEAN PATENT SPECIFICATION**

(45) Date of publication and mention
of the grant of the patent:
**22.07.2020 Bulletin 2020/30**

(51) Int Cl.:
*A23L 33/18* (2016.01)   *A61K 38/08* (2019.01)
*A61K 38/05* (2006.01)   *A61P 1/12* (2006.01)
*A61K 38/18* (2006.01)

(21) Application number: **15787999.0**

(22) Date of filing: **29.10.2015**

(86) International application number:
**PCT/EP2015/075154**

(87) International publication number:
**WO 2016/066758 (06.05.2016 Gazette 2016/18)**

(54) **MILK PROTEIN HYDROLYSATE FOR USE IN THE TREATMENT OF DIARRHOEA**

MILCHPROTEINHYDROLYSAT ZUR VERWENDUNG BEI DER BEHANDLUNG VON DURCHFALL

HYDROLYSAT DE PROTÉINE DU LAIT DESTINÉ À ÊTRE UTILISÉ DANS LE TRAITEMENT DE LA DIARRHÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.10.2014 EP 14191179**

(43) Date of publication of application:
**06.09.2017 Bulletin 2017/36**

(73) Proprietor: **Laboratorios Ordesa, S.l.
08830 Sant Boi de Llobregat (ES)**

(72) Inventors:
• **RIVERO URGELL, Montserrat**
  **E-08021 Barcelona (ES)**
• **RODRÍGUEZ-PALMERO SEUMA, María**
  **E-08029 Barcelona (ES)**
• **MORENO MUÑOZ, José Antonio**
  **E-08222 Terrassa (ES)**
• **PUIGJANER RIBA, Joaquim**
  **E-08794 Les Cabanyes (ES)**
• **GARCÍA CRUZ, Marc**
  **E-08211 Castellar del Vallès (ES)**
• **CIFUENTES ORJUELA, Gloria Clemencia**
  **E-08880 Cubelles (ES)**

(74) Representative: **ZBM Patents - Zea, Barlocci &
Markvardsen
Rambla Catalunya, 123
08008 Barcelona (ES)**

(56) References cited:
**WO-A1-2006/130200   WO-A1-2009/000899
US-A1- 2010 056 458   US-A1- 2011 263 505**

• **TESCHEMACHER HANSJORG ET AL: "Milk
protein-derived opioid receptor ligands",
BIOPOLYMERS, JOHN WILEY & SONS, INC, US,
vol. 43, no. 2, 1 January 1997 (1997-01-01), pages
99-117, XP009112638, ISSN: 0006-3525**
• **RAVINDER NAGPAL ET AL: "Bioactive peptides
derived from milk proteins and their health
beneficial potentials: an update", FOOD &
FUNCTION, vol. 2, no. 1, 1 January 2011
(2011-01-01), pages 18-27, XP055056004, ISSN:
2042-6496, DOI: 10.1039/c0fo00016g**
• **DHANDA; DHANDA ET AL.: "Functional
characterization and specific effects of peptides
on enzymatic activity of a DPP-III homologue from
goat brain", JOURNAL OF ENZYME INHIBITION
AND MEDICINAL CHEMISTRY, vol. 23, no. 2, 2008,
pages 174-181, XP009183284, cited in the
application**

Remarks:
The file contains technical information submitted after
the application was filed and not included in this
specification

SEQUENCE LISTING

<110> LABORATORIOS ORDESA, S.L.

<120> Milk protein hydrolysate for use in the treatment of diarrhoea

<130> P3036PC00

<150> EP14191179
<151> 2014-10-30

<160> 15

<170> PatentIn version 3.5

<210> 1
<211> 10
<212> PRT
<213> Bos taurus

<400> 1

```
Val Leu Gly Pro Val Arg Gly Pro Phe Pro
1               5               10
```

<210> 2
<211> 11
<212> PRT
<213> Bos taurus

<400> 2

```
Val Leu Gly Pro Val Arg Gly Pro Phe Pro Ile
1               5               10
```

<210> 3
<211> 224
<212> PRT
<213> Bos taurus

<400> 3

```
Met Lys Val Leu Ile Leu Ala Cys Leu Val Ala Leu Ala Leu Ala Arg
1               5               10              15

Glu Leu Glu Glu Leu Asn Val Pro Gly Glu Ile Val Glu Ser Leu Ser
            20              25              30

Ser Ser Glu Glu Ser Ile Thr Arg Ile Asn Lys Lys Ile Glu Lys Phe
            35              40              45

Gln Ser Glu Glu Gln Gln Gln Thr Glu Asp Glu Leu Gln Asp Lys Ile
        50              55              60

His Pro Phe Ala Gln Thr Gln Ser Leu Val Tyr Pro Phe Pro Gly Pro
65              70              75              80
```

```
Ile Pro Asn Ser Leu Pro Gln Asn Ile Pro Pro Leu Thr Gln Thr Pro
              85                  90                  95

Val Val Val Pro Pro Phe Leu Gln Pro Glu Val Met Gly Val Ser Lys
             100                 105                 110

Val Lys Glu Ala Met Ala Pro Lys His Lys Glu Met Pro Phe Pro Lys
             115                 120                 125

Tyr Pro Val Glu Pro Phe Thr Glu Ser Gln Ser Leu Thr Leu Thr Asp
    130                 135                 140

Val Glu Asn Leu His Leu Pro Leu Pro Leu Leu Gln Ser Trp Met His
145                 150                 155                 160

Gln Pro His Gln Pro Leu Pro Pro Thr Val Met Phe Pro Pro Gln Ser
             165                 170                 175

Val Leu Ser Leu Ser Gln Ser Lys Val Leu Pro Val Pro Gln Lys Ala
             180                 185                 190

Val Pro Tyr Pro Gln Arg Asp Met Pro Ile Gln Ala Phe Leu Leu Tyr
    195                 200                 205

Gln Glu Pro Val Leu Gly Pro Val Arg Gly Pro Phe Pro Ile Ile Val
    210                 215                 220
```

<210> 4
<211> 14
<212> PRT
<213> Bos taurus

<400> 4

```
Gln Glu Pro Val Leu Gly Pro Val Arg Gly Pro Phe Pro Ile
1               5                  10
```

<210> 5
<211> 13
<212> PRT
<213> Bos taurus

<400> 5

```
Gln Glu Pro Val Leu Gly Pro Val Arg Gly Pro Phe Pro
1               5                  10
```

<210> 6
<211> 17
<212> PRT
<213> Bos taurus

<400> 6

```
Tyr Gln Glu Pro Val Leu Gly Pro Val Arg Gly Pro Phe Pro Ile Ile
1               5               10              15

Val
```

<210> 7
<211> 15
<212> PRT
<213> Bos taurus

<400> 7

```
Tyr Gln Glu Pro Val Leu Gly Pro Val Arg Gly Pro Phe Pro Ile
1               5               10              15
```

<210> 8
<211> 14
<212> PRT
<213> Bos taurus

<400> 8

```
Tyr Gln Glu Pro Val Leu Gly Pro Val Arg Gly Pro Phe Pro
1               5               10
```

<210> 9
<211> 17
<212> PRT
<213> Bos taurus

<400> 9

```
Leu Tyr Gln Glu Pro Val Leu Gly Pro Val Arg Gly Pro Pro Ile Ile
1               5               10              15

Val
```

<210> 10
<211> 15
<212> PRT
<213> Bos taurus

<400> 10

```
Leu Tyr Gln Glu Pro Val Leu Gly Pro Val Arg Gly Pro Phe Pro
1               5               10              15
```

<210> 11
<211> 19
<212> PRT
<213> Bos taurus

<400> 11

```
        Leu Leu Tyr Gln Glu Pro Val Leu Gly Pro Val Arg Gly Pro Phe Pro
        1               5               10              15

        Ile Ile Val
```

<210> 12
<211> 16
<212> PRT
<213> Bos taurus

<400> 12

```
        Leu Leu Tyr Gln Glu Pro Val Leu Gly Pro Val Arg Gly Pro Phe Pro
        1               5               10              15
```

<210> 13
<211> 222
<212> PRT
<213> Bos taurus

<400> 13

```
        Met Lys Phe Phe Ile Phe Thr Cys Leu Leu Ala Val Ala Leu Ala Lys
        1               5               10              15

        Asn Thr Met Glu His Val Ser Ser Ser Glu Glu Ser Ile Ile Ser Gln
                    20              25              30

        Glu Thr Tyr Lys Gln Glu Lys Asn Met Ala Ile Asn Pro Ser Lys Glu
                    35              40              45

        Asn Leu Cys Ser Thr Phe Cys Lys Glu Val Val Arg Asn Ala Asn Glu
            50              55              60

        Glu Glu Tyr Ser Ile Gly Ser Ser Ser Glu Glu Ser Ala Glu Val Ala
        65              70              75              80

        Thr Glu Glu Val Lys Ile Thr Val Asp Asp Lys His Tyr Gln Lys Ala
                        85              90              95

        Leu Asn Glu Ile Asn Gln Phe Tyr Gln Lys Phe Pro Gln Tyr Leu Gln
                        100             105             110

        Tyr Leu Tyr Gln Gly Pro Ile Val Leu Asn Pro Trp Asp Gln Val Lys
                    115             120             125

        Arg Asn Ala Val Pro Ile Thr Pro Thr Leu Asn Arg Glu Gln Leu Ser
                130             135             140
```

```
Thr Ser Glu Glu Asn Ser Lys Lys Thr Val Asp Met Glu Ser Thr Glu
145             150             155             160

Val Phe Thr Lys Lys Thr Lys Leu Thr Glu Glu Glu Lys Asn Arg Leu
            165             170             175

Asn Phe Leu Lys Lys Ile Ser Gln Arg Tyr Gln Lys Phe Ala Leu Pro
            180             185             190

Gln Tyr Leu Lys Thr Val Tyr Gln His Gln Lys Ala Met Lys Pro Trp
            195             200             205

Ile Gln Pro Lys Thr Lys Val Ile Pro Tyr Val Arg Tyr Leu
    210             215             220
```

<210> 14
<211> 11
<212> PRT
<213> Bos taurus

<400> 14

```
Met His Gln Pro His Gln Pro Leu Pro Pro Thr
1               5               10
```

<210> 15
<211> 9
<212> PRT
<213> Artificial

<220>
<223> Peptide fluorogenic substrate of neprylisin

<220>
<221> MOD_RES
<222> (1)..(1)
<223> 7-Methoxycoumarinarginine

<220>
<221> MOD_RES
<222> (9)..(9)
<223> Dinitrophenyllysine (K-Dnp-OH)

<400> 15

```
Arg Pro Pro Gly Phe Ser Ala Phe Lys
1               5
```

**Description**

The present invention relates both to the field of nutrition and to the field of the treatment of diarrhoea. It relates to milk protein hydrolysates comprising peptides that act as potent inhibitors of intestine cell enzymes.

BACKGROUND ART

Diarrhoea or diarrhoea is defined by the World Health Organization as the condition of having three or more loose or liquid bowel movements per day.

The most common cause is gastroenteritis, generally caused by infection of pathogens such as Novovirus, Rotavirus, Salmonella, Sighella, among others.

It can also be one of the symptoms of malabsorption, which in turn is the consequence of some disorders in the small bowel, but also due to poor digestion from diseases of the pancreas. It is a common cause of death in developing countries and the second most common cause of infant deaths worldwide. The loss of fluids through diarrhoea can cause dehydration and electrolyte disturbances such as potassium deficiency or other salt imbalances. Diarrhoea is also a main problem of adult people that receives enteral feedings due to impaired bowel functions, surgery or the like.

There are several approaches for treating diarrhoea. One of the most extended is the use of oral rehydration solutions (ORS) with modest amounts of salts and zinc tablets. Another approach is the use of compositions comprising loperamide, and active agent that reduces intestine motility, thus reducing the frequency of defecation. One of the disadvantages of the compositions reducing motility is that the counterpart is constipation. In addition, a low motility of intestines (small and large bowel) may favour infection proliferation.

In the field of infant formulas and with the aim of making feasible the administration of a composition to treat diarrhoea in this type of sensitive population, there have been developed formulas with a proper balance of nucleotides and/or nucleosides, among other components.

Examples of these formulas are the compositions of US 5492899, wherein there is disclosed a method for controlling the stool consistency in children with a fatty acid ingredient.

Also the document EP2046149 discloses compositions to treat diarrhoea, in which a mixture of gangliosides, phospholipids, lactoferrin, and lipids modified with sialic acid is administered, together with other common ingredients in infant formulas.

Peptides obtained from casein and which have an anti-diarrhoeal activity are disclosed in e.g., Teschemacher H. et al, Biopoly 43: 99-117 (1997); Nagpal R et al, Food Funct. 2: 18-27 (2011); and WO 2009/000899.

Finally, another existing way for treating diarrhoea of any aetiology is by administration of neprilysin inhibitors.

Neprilysin (E.C. 3.4.24.11) is a zinc-dependent metalloprotease, synthesized as a membrane protein, whose ectodomain is released into the extracellular domain after the transport from Golgi to the cell surface. The enzyme is also named membrane metallo-endopeptidase, neutral endopeptidase (NEP), CD10 or common acute lymphoblastic leukemia antigen (CALLA). Neprilysin degrades several peptides and among those, it can cut the pentapeptides known as enkephalins, which are involved in the nociception processes. For this reason, neprilysin is also considered or categorized as an enkephalinase. Neprilysin is present in several tissues, such as in renal cells, lung cells and also in the intestine cells (in particular in the small bowel cells).

Inhibitors of neprilysin have been designed with the aim of developing analgesic and antihypertensive agents that act by preventing neprilysin's activity against signalling peptides such as enkephalins, substance P, endothelin, and atrial natriuretic factor.

An example of neprilysin inhibitor is thiorphan (($\pm$)-2-[(2-benzyl-3-sulfanyl-propanoyl)amino]acetic acid), the active metabolite of the racecadotril (acetorphan). Racecadotril is an antidiarrheal drug which acts as a peripherally acting enkephalinase inhibitor. Unlike other medications used to treat diarrhoea, which reduce intestinal motility, racecadotril has an antisecretory effect-it reduces the secretion of water and electrolytes into the intestine. Inhibition of neprilysin allows enkephalins to reach their receptors ($\delta$-opioid) that finally inhibit cAMP formation from ATP. Inhibition of cAMP formation avoids the secretion of water and salts into the intestine lumen.

Racecadotril is marketed in a stable granulated composition that has to be resuspended when administered. Even thought that racecadotril is the prodrug form of thiorphan (the active metabolite) developed to improve it, it still has poor water solubility. This implies the disadvantage of poor resuspension in the liquid vehicle with the loose of effective dose.

Therefore, there is still a need of other compositions for treating or preventing diarrhoea that while being safe, they are effective and do not have the above-mentioned problems.

SUMMARY OF THE INVENTION

The invention is defined by the claims. It is herewith provided milk protein hydrolysates with a high neprilysin inhibitory effect. These protein hydrolysates are obtainable by a method that gives rise to a peptide composition including short peptides (from 2 to 20 amino acids) that makes it soluble in many liquids and easy to be mixed with other ingredients. Hence, the hydrolysate supposes a feasible product to treat and/or prevent diarrhoea of any aetiology, in particular by means of inhibition of the enzyme neprilysin. This aims facing diarrhoea in which neprilysin is highly active.

Thus, in a first aspect the invention relates to milk protein hydrolysates, for use in the prevention and/or treatment of diarrhoea, wherein the protein hydrolysates are capable of inhibiting neprilysin enzyme with an IC50 from 0.005 to 0.600 mg/ml, and which are obtainable by a method comprising the steps of:

(a) adding to a composition comprising milk protein that comprises milk casein selected from the group consisting of beta-casein, alpha-casein, and mixtures thereof, in a total milk protein concentration from 3 % to 20 % weight/volume (w/v), at least a protease system that is active at an optimal pH from 5.5 to 8.5, said protease system comprising a protease selected from the group consisting of thermolysin, or a mixture of neutral protease and alkaline protease; and
(b) incubating at a constant pH from 6 to 8 and to a temperature from 45 °C to 55 °C the mixture of (a) to hydrolysate the milk protein until a degree of hydrolysis (DH) of the composition is from 1.0 to 40.0 %;

wherein DH is calculated by means of the following formula:

$$DH = B \times Nb \times (1/a) \times 1/MP \times 1/Htot \times 100;$$

wherein

B is the volume in millilitres (ml) of consumed base used for titrating released amino groups during hydrolysis of the composition comprising milk protein,
Nb is the normality of the base used for titrating,
1/a is the average degree of dissociation of the amino groups related with the pK of said amino groups at a particular pH and temperature, MP is the amount in grams of the composition comprising milk protein to be incubated with the protease system, and
Htot in milliequivalents per g (meq/g) is the sum of the millimoles of individual amino acids per gram of protein associated with the composition comprising milk protein; and

wherein the IC50 is the concentration of the hydrolysate that inhibits 50 % of the neprilysin enzyme activity, said IC50 determined by a fluorogenic enzymatic assay with a fluorescence excitation wavelength of 320 nm and with a fluorescence emission wavelength of 405 nm, the assay performed at 37 °C with dilutions of hydrolysate samples from no dilution to a dilution of at least 1/100 in water, in order to obtain a curve of percentage of inhibition of the enzyme per assayed hydrolysate dilution.

The specific combination of enzymes for the hydrolysis of the specific milk protein allows obtaining some bioactive peptides, that when forming part of the hydrolysates provide inhibition of neprilysin with an IC50 from of 0.005 mg/ml to 0.600 mg/ml.

As will be depicted below, some of the milk protein hydrolysates, in particular milk casein hydrolysates contain as active peptides, peptides from 10 to 20 amino acids and comprising the amino acid sequence VLGPVRGPFP (SEQ ID NO: 1), and peptides of formula (I) $X^1W$, wherein $X^1$ is selected from leucine (L) and isoleucine (I).

In a parallel analysis, the inventors determined that the inhibitory capacity (IC50) of the peptide LW was of $2.69 \times 10^{-2}$ mg/ml (84.76 μM). In the same analytical conditions Thiorphan shows a value of IC50 of $3.08 \times 10^{-5}$ mg/ml (0.1214 μM).

Thus, although the IC50 of Thiorphan is lower, the values of the obtained milk protein hydrolysates of the invention are low enough and imply the advantage of being compositions without the possible adverse effects of a drug. In the particular case of Thiorphan, one of the disclosed adverse effects is the headache (10 % of cases).

Therefore, the inventors provide milk protein hydrolysates that may be good candidates for substituting active principles of chemical synthesis. A similar situation is already known in the case of inhibitors of the enzyme Angiotensin Converting Enzymes (ACE), in which the synthetic drug captopril (antihypertensive with an IC50 similar to that of Thiorphan) is being substituted by some peptides (IPP, VPP) with the capacity of inhibiting ACE with an IC50 of 5-9 μM. Other ACE inhibitory peptides have IC50s up to 900 μM.

Yet another aspect is a milk protein hydrolysate for use in the treatment and/or prevention of diarrhoea, wherein said milk protein hydrolysate comprises a peptide from 10 to 20 amino acids and comprising the amino acid sequence VLGPVRGPFP (SEQ ID NO: 1), and comprises a peptide of formula (I) $X^1W$, wherein $X^1$ is selected from leucine (L) and isoleucine (I), said hydrolysate obtainable by a method comprising the steps of:

(a) adding to a composition comprising milk alpha-casein and milk beta-casein in a total protein concentration from 3 % to 20 % weight/volume (w/v), at least a protease system that is active at an optimal pH from 5.5 to 8.5, said protease system comprising a protease selected from the group consisting of a mixture of neutral protease and alkaline protease, or thermolysin; and

(b) incubating at a constant pH from 6 to 8 and to a temperature from 45 ° C to 55 °C the mixture of (a) to hydrolysate the milk protein until a degree of hydrolysis (DH) of the composition is from 1.0 to 40.0 %;

wherein DH is calculated as above exposed, and wherein the hydrolysate is capable of inhibiting neprilysin enzyme with an IC50 from 0.005 to 0.600 mg/ml, being the IC50 defined and determined also as above exposed.

In another aspect is a milk hydrolysate capable of inhibiting neprilysin enzyme with an IC50 from 0.005 tom 0.600 mg/ml, for use in the prevention and/ or treatment of diarrhoea, wherein said milk protein hydrolysate is obtainable by a method comprising the steps of:

(a) adding to a composition comprising milk protein, said milk protein comprising milk beta-casein and a total protein concentration from 3% to 20% weight/volume (w/v), at least a mixture of neutral protease and alkaline protease with a ratio of both enzymes from 5:1 to 1:5, the amount of neutral protease per mess unit of total protein in the milk protein composition from 2x 10$^{-3}$ AU/g to 2 x 10$^{-1}$ AU/g and the amount of alkaline protease from 6 x 10$^{-3}$ AU/g to 6 x 10$^{-1}$ AU/g; and

(b) incubating at a constant pH from 6 to 8 and to a temperature from 45°C to 55°C the mixture of (a) to hydrolyse the milk protein until a degree of hydrolysis (DH) of ther composition is from 5.0 to 40.0%; wherein the milk protein hydrolysate after step (b) comprises the milk beta-casein peptide from 10 to 20 amino acids and comprising the amino acid sequence VLGPVRGPFP (SEQ ID NO: 1), and a milk beta-casein peptide from 11 to 17 amino acids and comprising the sequence MHQPHQPLPPT (SEQ ID NO: 14).

The milk protein hydrolysates according to the invention include a peptide pattern (peptide composition) derived from milk casein hydrolysis, but, also peptides derived from other proteins present in the milk protein composition comprising said casein, such as whey protein of the milk.

These milk protein hydrolysates of the invention or any composition comprising them, are for use in the prevention and/or treatment of diarrhoea.

The present invention may be used in a method for the treatment or prevention of diarrhoea comprising administering a therapeutically effective amount of any of the milk protein hydrolysates as defined above, together with pharmaceutically acceptable excipients or carriers, in a subject in need thereof, including a human.

In addition, the milk protein hydrolysates of the invention can be administered as well-mixed ingredients in compositions and foods (i.e. infant formulas).

Thus, another aspect is an edible composition comprising the milk protein hydrolysate as defined above. These compositions are for use in the treatment and/or prevention of diarrhoea in an animal, including a human.

Being effective against diarrhoea or allowing alleviating diarrhoea, the milk protein hydrolysates can be administered as medicaments. Thus, particular compositions comprising them are pharmaceutical compositions with a therapeutically effective amount of the protein hydrolysate as defined above, together with appropriate amounts of pharmaceutical acceptable excipients and/or carriers.

BRIEF DESCRIPTION OF THE DRAWINGS

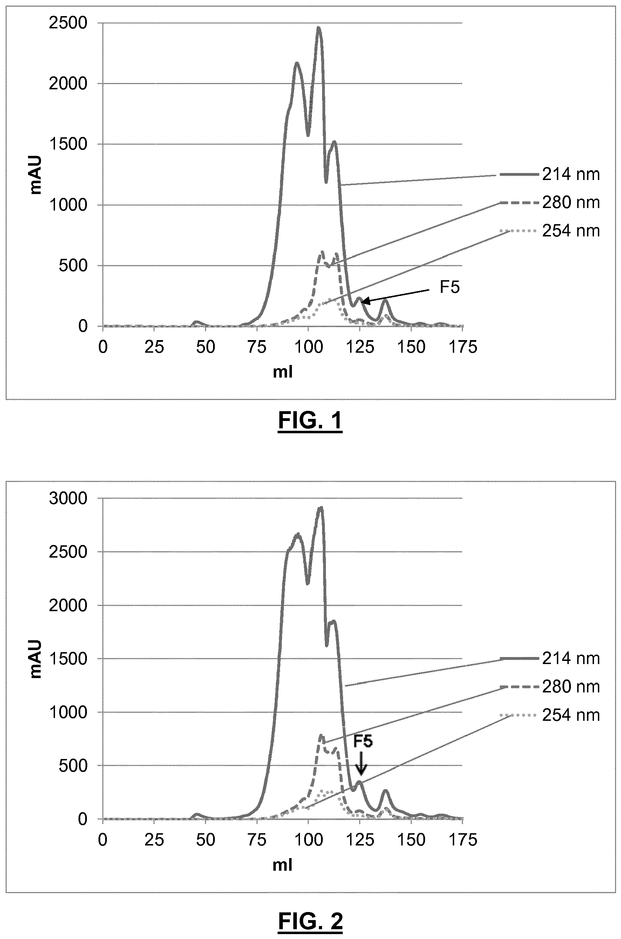

FIG. 1, relating to Example 2, is a size exclusion chromatogram showing absorbance in arbitrary units (mA.U.) and measured at 214 nm, 280 nm, and 254 nm of a milk protein hydrolysate of the invention with a DH = 21.09 % . The X-axis shows the volume (ml) of eluent passed-through. Column: Superdex 30 pg, GE Healthcare. Fraction 5 (F5) is a fraction of the hydrolysate comprising the bioactive peptide LW.

FIG. 2, relating also to Example 2, is another size exclusion chromatogram showing absorbance in arbitrary units (mA.U.) and measured at 214 nm, 280 nm, and 254 nm of a milk protein hydrolysate of the invention with a DH = 33.14 %. The X-axis shows the volume (ml) of eluent passed-through. Column: Superdex 30 pg, GE Healthcare. Fraction 5 (F5) is a fraction of the hydrolysate comprising the bioactive peptide LW.

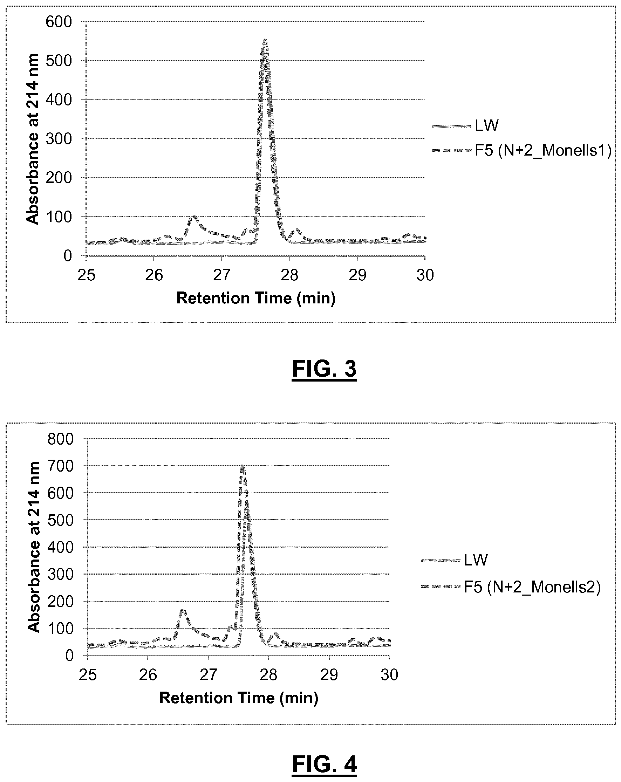

FIG. 3, relating to Example 2, shows a Reverse Phase- High Performance Liquid Chromatography (RP-HPLC) chromatogram of F5 isolated from a milk protein hydrolysate with a DH = 21.09 % (dashed line); and the chromatogram of a control (C, LW) including 0.04 mg/ml of the pure peptide LW (continuous line). C-18 column. Solvent system included solvent A (water-TFA 1000:1), and solvent B (acetonitrile-TFA: 1000:1). Absorbance at 214 nm is indicated in arbitrary units (A.U.) versus the retention time in minutes (min).

FIG. 4, also relating to Example 2, shows a Reverse Phase- High Performance Liquid Chromatography (RP-HPLC) chromatogram of F5 isolated from a milk protein hydrolysate with a DH = 33.14 % (dashed line); and the chromatogram of a control (C, LW) including 0.04 mg/ml of the pure peptide LW (continuous line). C-18 column. Solvent system included solvent A (water-TFA 1000:1), and solvent B (acetonitrile-TFA: 1000:1). Absorbance at 214 nm is indicated in arbitrary units (A.U.) versus the retention time in minutes (min).

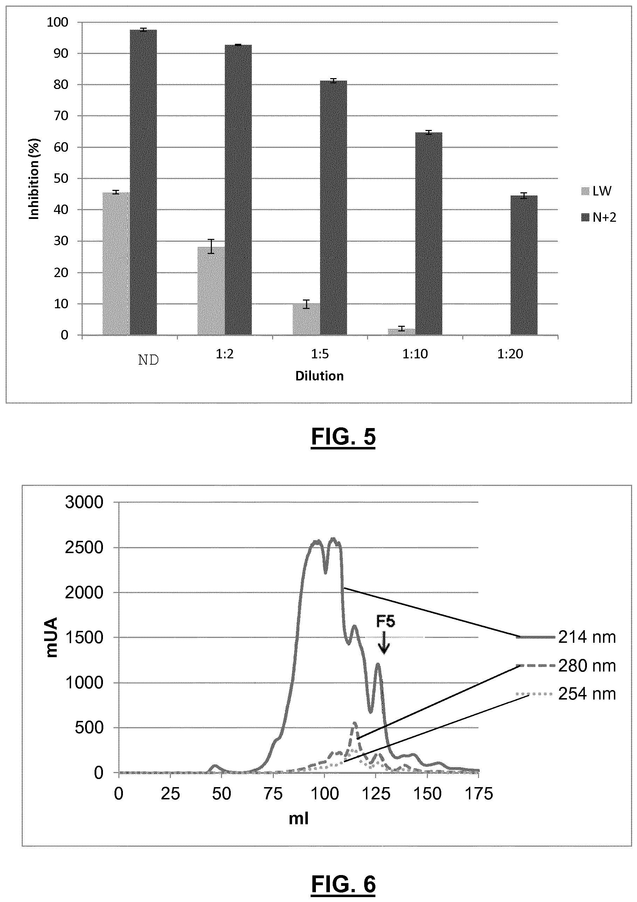

FIG. 5, relating to Example 2, is a bar diagram showing the percentage of neprilysin inhibition (%) (Y-axis) of different particular dilutions (X-axis) of a milk protein hydrolysate of the invention N+2 (right bars in each dilution) and of a composition with pure LW at the same concentration as in the hydrolysate (left bars in each dilution). ND means no dilution.

FIG. 6, relating to Example 2, shows as in FIGs. 1 and 2, the results of a size exclusion chromatogram showing absorbance in arbitrary units (mA.U.) and measured at 214 nm, 280 nm, and 254 nm of a milk protein hydrolysate of the invention with a DH = 5.00 %. The X-axis shows the volume (ml) of eluent passed-through. Column: Superdex 30 pg, GE Healthcare. Fraction 5 (F5) is a fraction of the hydrolysate comprising the bioactive peptide LW.

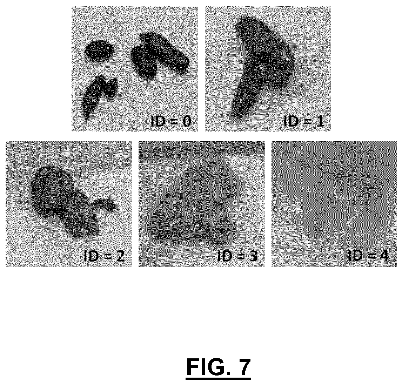

FIG. 7 corresponds to pictures of defecation types produced by healthy rats (control) not receiving castor oil (ID=0), and produced by rats with induced acute diarrhoea (ID= 1-4).

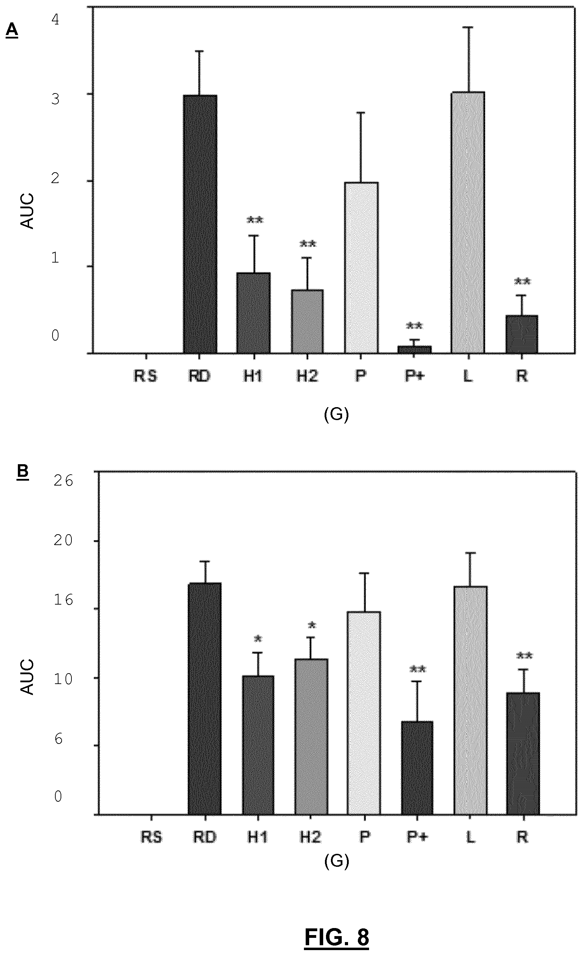

FIG. 8 is a graphic showing in panel the severity of diarrhoeic process (expressed as area under the curve, AUC, of the diarrhoeic index measured for a specified period of time) in each group (G, X-axis). FIG. 8A shows results for a period from 0 to 3 h PI, and FIG. 8B shows results for a period from 0 to 8 h PI. Group identification is as illustrated in Table 6 of the examples.

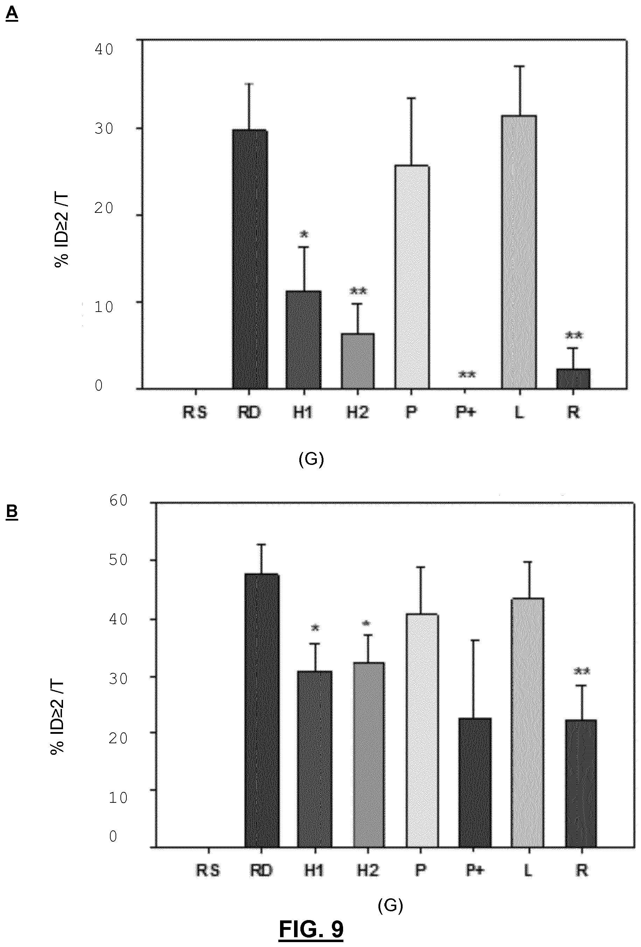

FIG. 9, relating to Example 7, is a graphic showing the percentage of diarrhoeic defecations in respect of the total (% ID≥2 / T) per group (G) during a specified time period. FIG. 9A shows results for a period from 0 to 3 h PI, and FIG. 9B shows results for a period from 0 to 8 h PI. Group identification is as illustrated in Table 6 of the examples.

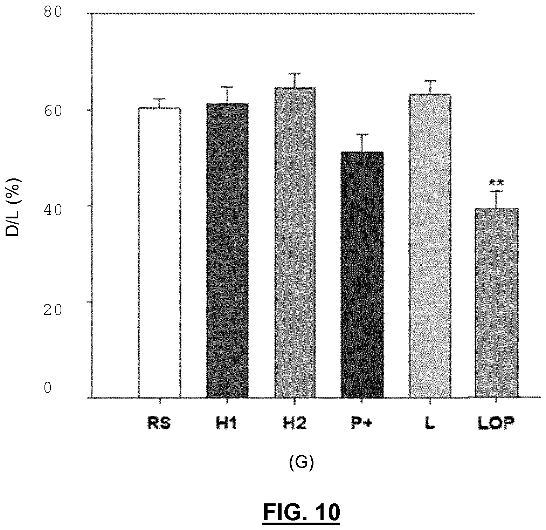

FIG. 10, relating to Example 8, is a bar diagram showing for each assayed group (G) the percentage of distance of administered activated charcoal in the total length of small intestine (D/L (%).Group identification is as illustrated in Table 6 of the examples with the exception that no R group is present and that a new group (LOP) appears, corresponding to an administered dose of Loperamide of 2 mg/Kg of animal.

DETAILED DESCRIPTION OF THE INVENTION

The invention is defined by the claims.

If not indicated to the contrary all the terms and concepts appearing in this description must be understood as having the common accepted meaning for the skilled man in the art. For easy understanding, the following definitions are included.

In the sense of the present invention "an optimal pH or optimal pH range of an enzyme" is to be understood the pH value or range, in which its protein conformation (due to electrical charges) is the best to perform catalytic activity. It is a widely accepted term in enzymology.

The parameter IC50, also named "half maximal inhibitory concentration (IC50)" is a measure of the effectiveness of a substance in inhibiting a specific biological or biochemical function. In this particular case, it is the concentration of hydrolysate that inhibits 50 % of the enzyme (i.e. neprilysin) activity. It is usually determined by testing several concentrations of a particular inhibitor (hydrolysate) interfering in the enzymatic assay. A curve (dose or concentration-inhibition) is plotted in a so-called curve of percentage of inhibition per assayed concentration of inhibitor (hydrolysate). IC50 is the concentration at which the curve passes through the 50% inhibition level.

In the sense of the present invention an edible composition, also termed a food product, includes a milk product, a baby cereal, a yogurt, a curd, a cheese (e.g. quark, cream, processed, soft and hard), a fermented milk, a milk powder, a milk based fermented product, an ice-cream, a fermented cereal based product, a milk based powder, a beverage, a dressing, and a pet food. Examples of other food products are meat products (e.g. liver paste, frankfurter and salami sausages or meat spreads), chocolate spreads, fillings (e.g. truffle, cream) and frostings, chocolate, confectionery (e.g. caramel, fondants or toffee), baked goods (cakes, pastries), sauces and soups, fruit juices and coffee whiteners. However, the term "food product" is used herein in its broadest meaning, including any type of product, in any form of presentation, which can be ingested by an animal.

"Nutritional compositions" are edible compositions including dietary supplements, oral rehydration solutions (ORS), food additives, baby cereals and infant formulas. Dietary supplements intend to supply nutrients, (vitamins, minerals, fatty acids or amino acids) that are missing or not consumed in sufficient quantity in a person's diet (infants, pregnant women, elderly people, etc). Infant formulas relate to manufactured food designed and marketed for feeding to babies and infants under 12 months of age, usually prepared for bottle-feeding or cup-feeding from powder (mixed with water) or liquid (with or without additional water). The U.S. Federal Food, Drug, and Cosmetic Act (FFDCA) defines infant formula as "a food which purports to be or is represented for special dietary use solely as a food for infants by reason of its simulation of human milk or its suitability as a complete or partial substitute for human milk.

For "milk" is to be understood the white liquid produced by the mammary glands of mammals. It is the primary source of nutrition for young mammals before they are able to digest other types of food. Normal bovine milk contains 30-35 grams of protein per liter of which about 80% is arranged in casein micelles. The largest structures in the fluid portion of the milk are "casein micelles" aggregates of several thousand protein molecules with superficial resemblance to a surfactant micelle, bonded with the help of nanometer-scale particles of calcium phosphate. Each casein micelle is roughly spherical and about a tenth of a micrometer across. There are four different types of casein proteins: alpha1 ($\alpha$s1)-, alpha2 ($\alpha$s2)-, beta ($\beta$)-, and kappa ($\kappa$)-caseins. When in the present invention the term alpha casein is employed it relates to both ($\alpha$s1)- and ($\alpha$s2)-. Collectively, all these caseins make up around 76-86% by weight of the protein in milk. Milk also includes lipids, vitamins, minerals, salts and several carbohydrates.

For "whey protein" or "whey" it is to be understood the collection of globular proteins isolated from whey, a by-product of cheese manufactured from cow's milk. The protein in cow's milk is 20% whey protein and 80% casein protein, whereas the protein in human milk is 60% whey and 40% casein. The protein fraction in whey constitutes approximately 10% of the total dry solids in whey. This protein is typically a mixture of beta-lactoglobulin (~65%), alpha-lactalbumin (~25%), bovine serum albumin (~8%, and immunoglobulins.

The term "pharmaceutically acceptable" as used herein refers to compounds, materials, compositions, and/or dosage forms which are, within the scope of sound medical judgement, suitable for use in contact with the tissues of a subject (either a human or non-human animal) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical texts.

The term "pharmaceutically effective amount" as used herein, means an amount of an active agent high enough to deliver the desired benefit, but low enough to avoid serious side effects within the scope of medical judgment.

According to the invention, the protein hydrolysate for use in the prevention and/or treatment of diarrhoea is obtainable by a method carried out with a milk protein composition that comprises at least milk casein selected from the group consisting of beta-casein, alpha-casein, and mixtures of these caseins.

When the composition comprising milk protein comprises or consists in a milk casein hydrolysate, the method is carried out by:

(a) adding to a composition comprising milk casein selected from the group consisting of beta-casein, alpha-casein, and mixtures thereof, and a total protein concentration from 3 % to 20 % weight/volume (w/v), at least a protease system having an optimal activity pH from 5.5 to 8.5, said protease system comprising a protease selected from the group consisting of a mixture of neutral protease and alkaline protease, or thermolysin; and
(b) incubating at a constant pH from 6 to 8 and to a temperature from 45 °C to 55 °C the mixture of (a) to hydrolysate the milk beta-and/or alpha-casein until a degree of hydrolysis (DH) of the composition is from 1.0 to 40 %.

Since according to the invention at least milk casein protein is used, the method provides a milk casein hydrolysate in which:

(i) when the composition comprising milk casein of step (a) comprises milk beta-casein, then the milk casein hydrolysate after step (b) comprises a peptide from 10 to 20 amino acids and comprising the amino acid sequence VLGPVRGPFP (SEQ ID NO: 1);

(ii) when the composition comprising milk casein of step (a) comprises milk alpha-casein, then the milk casein hydrolysate after step (b) comprises a peptide of formula (I) $X^1W$, wherein $X^1$ is selected from leucine (L) and isoleucine (I); and

(iii) when the composition comprising milk casein of step (a) comprises a mixture of beta-casein and alpha-casein, then the milk casein hydrolysate after step (b) comprises a mixture of the peptides as disclosed in (i) and (ii).

In a particular embodiment, the composition comprising milk protein consists in milk casein and in turn it comprises milk beta-casein, kappa-casein and alpha-casein.

As will be illustrated in the examples bellow, the invention provides a milk protein hydrolysate, namely a casein hydrolysate, with interesting properties. Although any composition comprising milk casein selected from the group consisting of beta-casein, kappa-casein, alpha-casein and mixtures thereof may be used in for obtaining the hydrolysate for use according to the invention, , in a particular embodiment the composition is *Bos Taurus* (cow) milk. Besides, *Capra hircus* (goat) milk and *Ovis aries* (sheep) milk can also be used, since-beta and alpha-casein of all these species have amino acid sequences with an identity of at least 85 % between cow and goat, and of at least 95 % between the casein proteins of goat and sheep. The identity is defined by the algorithm BLASTP disclosed in Altschul, S. F., et. al. "Gapped BLAST and PSI-BLAST: a new generation of proteína database search programms", Nucleic Acids Research - 1997, Vol. No. 25, pp.: 3389 - 3402, and NCBI http://www.ncbi.nlm.nih.gov/BLAST.

In another particular embodiment of the first aspect of the invention, optionally in combination with any of the embodiments above or below, the milk protein composition at initial of step (a) has a total protein concentration from 3 % to 15 % weight/volume (w/v). In a more particular embodiment the total protein concentration is from 6 % to 10 % (w/v). More particularly, the total protein concentration is 6.8 % (w/v). These protein concentrations are applicable in case the composition comprises whey protein and milk casein, as well as when only milk casein is present as protein composition to be hydrolysed. In the particular case of using only a milk casein composition comprising alpha-casein, beta-casein, kappa-casein or mixtures thereof, the final protein concentration to be hydrolysed is also in the same ranges indicated above.

Yet in another particular embodiment of the first aspect of the invention, optionally in combination of any of the embodiments below or above, the IC50 for neprilysin inhibition of the obtainable milk protein hydrolysate is from 0.020 mg/ml to 0.500 mg/ml. In a more particular embodiment from 0.020 mg/ml to 0.300 mg/ml. IC50 calculated as indicated in the Examples.

The protein hydrolysate for use according to the invention is in particular obtainable by a method carried out with a mixture of neutral protease and alkaline protease as the protease system.

In a more particular embodiment, optionally in combination with any embodiment above or below, when the protease system is a mixture of neutral protease and alkaline protease, in step a) the amount of enzyme expressed as Anson Units (AU) of added neutral protease per mass unit (in grams) of total protein in the milk protein composition is from $2 \times 10^{-3}$ AU/g to $2 \times 10^{-1}$ AU/g, and the amount of added alkaline protease per mass unit (in grams) of total protein in the milk protein composition is from $6 \times 10^{-3}$ AU/g to $6 \times 10^{-1}$ AU/g.

In a preferred embodiment, the amount of neutral protease is from $1.00 \times 10^{-2}$ AU/g to $2 \times 10^{-2}$ AU/g, preferably $1.47 \times 10^{-2}$ AU/g.

In another preferred embodiment, optionally in combination with any embodiment above or below, the amount of added alkaline protease is from $3 \times 10^{-2}$ AU/g to $3 \times 10^{-1}$ AU/g, preferably $4.41 \times 10^{-2}$ AU/g.

The enzyme activity is reported in Anson units. One Anson unit is defined as the amount of enzyme which, under specified conditions, digests urea-denatured hemoglobin at an initial rate such that there is liberated an amount of Trichloroacetic -soluble product per minute which gives the same color with Folin-Ciocalteu Phenol reagent as one milliequivalent of tyrosine at 25 °C at pH 7.50.

When the mixture of neutral protease and alkaline protease is used in the method for obtaining the hydrolysate for use according to the invention, the ratio of neutral protease:alkaline protease is from 5:1 to 1:5. A more particular ratio of enzyme amounts is 1:1.

It is also of particular interest a method of producing a milk protein hydrolysate, and the protein hydrolysate so obtainable, capable of inhibiting neprilysin enzyme with an IC50 from 0.005 to 0.600 mg/ml, the method comprising the steps of:

(a) adding to a composition comprising milk protein, said milk protein comprising milk casein and a total protein concentration from 3 % to 20 % weight/volume (w/v), at least a mixture of neutral protease and alkaline protease with a ratio of both enzymes from 5:1 to 1:5, and with an added amount of each of the enzymes per mass unit of total protein in the milk protein composition within the ranges indicated above per each enzyme; and

(b) incubating at a constant pH from 6 to 8 and to a temperature from 45 °C to 55 °C the mixture of (a) to hydrolysate the milk protein until a degree of hydrolysis (DH) of the composition is from 5.0 to 40.0 %; wherein

(i) when the milk protein composition comprising milk casein of step (a) comprises milk beta-casein, then the milk protein hydrolysate after step (b) comprises a milk beta-casein peptide from 10 to 20 amino acids and comprising the amino acid sequence VLGPVRGPFP (SEQ ID NO: 1), and a milk beta-casein peptide from 11 to 17 amino acids and comprising the sequence MHQPHQPLPPT (SEQ ID NO: 14);

(ii) when the composition comprising milk casein of step (a) comprises milk alpha-casein, then the milk protein hydrolysate after step (b) comprises a milk alpha-casein peptide of formula (I) $X^1W$, wherein $X^1$ is selected from leucine (L) and isoleucine (I); and

(iii) when the composition comprising milk casein of step (a) comprises a mixture of beta-casein and alpha-casein, then the milk protein hydrolysate after step (b) comprises a mixture of the peptides as disclosed in (i) and (ii).

The peptides of formula (I), and more in particular the peptide LW, and the peptides from 10 to 20 amino acids and comprising the amino acid sequence VLGPVRGPFP (SEQ ID NO: 1) are potent inhibitors of neprilysin as will be shown in the examples.

Additionally, the milk beta-casein peptides from 11 to 17 amino acids and comprising SEQ ID NO: 14 have been previously reported as potent inhibitor of rotavirus infectivity, one of the agents commonly originating the diarrhoea (PCT publication WO 2009000899 (Laboratorios Ordesa, S.L.).

These compounds of formula (I) as well as the peptides comprising SEQ ID NO: 1 can be used in the treatment of diarrhoea by means of inhibition of neprilysin, either alone or supplied in the form of milk protein hydrolysates comprising particularly milk casein hydrolysates.

The dipeptide LW is known, for example, from the document of Dhanda et al., "Functional characterization and specific effects of peptides on enzymatic activity of a DPP-III homologue from goat brain" Journal of Enzyme Inhibition and Medicinal Chemistry -2008, Vol. No. 23(2), pp.: 174-181. Dhanda et al., suggest the use of the peptide as inhibitor of the particular enkephalinase DPP-III.

Peptides comprising from 10 to 20 amino acids and comprising the amino acid sequence VLGPVRGPFP (SEQ ID NO: 1) are obtainable by hydrolysis of beta-casein from milk of a mammal selected from *Bos Taurus, Capra Hircus* and *Bos aries.*

In a more particular embodiment of the milk protein hydrolysate for use according to invention, it is obtainable by a method in which when a mixture of neutral protease and alkaline protease is used as indicated above, the degree of hydrolysis (DH) of the milk protein hydrolysate obtained by the method is from 20% to 35 %, and the milk protein hydrolysate obtainable comprises a milk beta-casein peptide from 10 to 20 amino acids and comprising the amino acid sequence VLGPVRGPFP (SEQ ID NO:1), more particularly a peptide consisting in SEQ ID NO: 1; a milk beta-casein peptide from 11 to 17 amino acids and comprising SEQ ID NO: 14, more particularly a peptide consisting in SEQ ID NO: 14; and a milk alpha-casein peptide of formula (I) $X^1W$, wherein $X^1$ is L. This particular milk protein hydrolysate is obtainable when the milk protein composition of step (a) comprises both beta-casein and alpha-casein, being preferred a cow milk protein composition as starting material. The milk protein hydrolysate obtainable by this particular method of the invention is capable of inhibiting neprilysin with an IC50 from 0.020 to 0.500 mg/ml.

In another particular embodiment of the milk protein hydrolysate for use according to invention, it is obtainable by a method in which the protease system is thermolysin. In a more particular embodiment, when the protease system is thermolysin, the milk protein composition of step (a) comprises at least milk beta-casein, particularly cow milk-beta casein; the degree of hydrolysis (DH) of the milk protein hydrolysate obtainable from the method is from 5% to 15%, particularly 10 %; and the resulting milk protein hydrolysate comprises a milk beta-casein peptide from 10 to 20 amino acids and comprising the amino acid sequence VLGPVRGPFP (SEQ ID NO:1), more particularly a peptide consisting in SEQ ID NO: 1. The milk protein hydrolysate obtainable by this particular method of the invention is capable of inhibiting neprilysin with an IC50 from 0.30 to 0.40 mg/ml.

In another particular embodiment, when the protease system is thermolysin, the milk protein composition of step (a) comprises at least milk beta-casein and milk alpha-casein, particularly cow milk beta- and alpha-casein; the degree of hydrolysis (DH) of the milk protein hydrolysate obtainable from the method is from 5% to 15%, particularly 10 %; and the resulting milk protein hydrolysate comprises a milk beta-casein peptide from 10 to 20 amino acids and comprising the amino acid sequence VLGPVRGPFP (SEQ ID NO:1), more particularly a peptide consisting in SEQ ID NO: 1; and a milk alpha-casein peptide of formula (I) $X^1W$, wherein $X^1$ is selected from leucine (L) and isoleucine (I), more particularly a peptide consisting in LW.

In a particular embodiment, optionally in combination with any of the embodiments above or below, the milk protein hydrolysate for use according to invention, it is obtainable by a method carried out at constant pH from 6 to 8. In a more particular embodiment of the method, pH is maintained at 7. Indeed, control of pH is important for maintaining the protease system at optimal catalytic conditions. It is widely known that each enzyme has an optimal pH or optimal pH range, in which its protein conformation (due to electrical charges) is the best to perform catalytic activity. In the method of the invention, the protease system works at an optimal pH range from 5.5 to 8.5. For this reason, pH of the hydrolysis reaction is maintained near 7.

In another particular embodiment, optionally in combination with any embodiment above or below, the milk protein hydrolysate for use according to invention, it is obtainable by method that further comprises a step of filtering the composition of (b) through a filter or mesh with a pore or cut-off of peptide molecular weight equal or lower than 3 kDa, particularly equal or lower than 2 kDa. This means that all the peptides in the milk protein hydrolysate of step (b) with a molecular weight equal or lower than 3 kDa will be retrieved from the filtrate. With this filtering step, there are separated those peptides with a molecular weight equal or lower than 3 kDa. The filtrate obtainable is a milk protein hydrolysate with low allergenic properties. In addition, with these filtrated hydrolysates lower IC50 values for neprilysin inhibition are obtained.

Yet in another particular embodiment, the method comprises the step of filtering as above indicated and purifying (separating) the peptides with a molecular weight equal or lower than 3 kDa by means of chromatography, such as), High Performance Liquid Chromatography (HPLC), and Fast Protein Liquid Chromatography (FPLC).

With the method steps disclosed above, several active milk protein hydrolysates (and particularly milk casein hydrolysates) can be obtained, said hydrolysates capable of inhibiting neprilysin enzyme.

Thus, in one aspect the invention relates to a milk protein hydrolysate for use in the prevention and/or treatment of diarrhoea, wherein the protein hydrolysate is capable of inhibiting neprilysin enzyme with an IC50 from 0.005 to 0.600 mg/ml, and it is obtainable by a method comprising the steps of:

(a) adding to a composition comprising milk protein that comprises milk casein selected from the group consisting of beta-casein, alpha-casein, and mixtures thereof, in a total milk protein concentration from 3 % to 20 % weight/volume (w/v), at least a protease system that is active at an optimal pH from 5.5 to 8.5, said protease system comprising a protease selected from the group consisting of a mixture of neutral protease and alkaline protease, or thermolysin;
(b) incubating at a constant pH from 6 to 8 and to a temperature from 45 ° C to 55 °C the mixture of (a) to hydrolysate the milk protein until a degree of hydrolysis (DH) of the composition is from 1.0 to 40.0 %;

wherein DH is calculated by means of the following formula:

$$DH = B \times Nb \times (1/a) \times 1/MP \times 1/Htot \times 100;$$

wherein

B is the volume in millilitres (ml) of consumed base used for titrating released amino groups during hydrolysis of the composition comprising milk protein,
Nb is the normality of the base used for titrating,
1/a is the average degree of dissociation of the amino groups related with the pK of said amino groups at a particular pH and temperature, MP is the amount in grams of the composition comprising milk protein to be incubated with the protease system, and
Htot in milliequivalents per g (meq/g) is the sum of the millimoles of individual amino acids per gram of protein associated with the composition comprising milk protein; and

wherein the IC50 is the concentration of the hydrolysate that inhibits 50 % of the neprilysin enzyme activity, said IC50 determined by a fluorogenic enzymatic assay with a fluorescence excitation wavelength of 320 nm and with a fluorescence emission wavelength of 405 nm, the assay performed at 37 °C with dilutions of hydrolysate samples from no dilution to a dilution of at least 1/100 in water, in order to obtain a curve of percentage of inhibition of the enzyme per assayed hydrolysate dilution.

In a particular aspect defined by a milk protein hydrolysate that comprises a peptide from 10 to 20 amino acids and comprising the amino acid sequence VLGPVRGPFP (SEQ ID NO: 1), said protein hydrolysate is obtainable by a method in which the protease system of step (a) is a mixture of neutral protease and alkaline protease.

In yet a more particular embodiment, the invention provides a milk protein hydrolysate that comprises a peptide from 10 to 20 amino acids and comprising the amino acid sequence VLGPVRGPFP (SEQ ID NO: 1), the protein hydrolysate is capable of inhibiting neprilysin enzyme with an IC50 from 0.005 to 0.600 mg/ml, and it is obtainable by a method comprising the steps of:

(a) adding to a composition comprising milk protein, said milk protein comprising milk beta-casein and a total protein concentration from 3 % to 20 % weight/volume (w/v), at least a mixture of neutral protease and alkaline protease with a ratio of both enzymes from 5:1 to 1:5, the amount of neutral protease per mass unit of total protein in the milk protein composition from $2 \times 10^{-3}$ AU/g to $2 \times 10^{-1}$ AU/g and the amount of alkaline protease from $6 \times 10^{-3}$ AU/g to $6 \times 10^{-1}$ AU/g; and
(b) incubating at a constant pH from 6 to 8 and to a temperature from 45 ° C to 55 °C the mixture of (a) to hydrolysate the milk protein until a degree of hydrolysis (DH) of the composition is from 5.0 to 40.0 %; wherein the milk protein hydrolysate after step (b) comprises the milk beta-casein peptide from 10 to 20 amino acids and comprising the amino acid sequence VLGPVRGPFP (SEQ ID NO: 1), and a milk beta-casein peptide from 11 to 17 amino acids and comprising the sequence MHQPHQPLPPT (SEQ ID NO: 14).

In a particular embodiment of the aspect defined by a milk protein hydrolysate that comprises a peptide of formula (I) X$^1$W, wherein X$^1$ is selected from leucine (L) and isoleucine (I), said hydrolysate is obtainable by a method comprising as protease system in step (a) a mixture of neutral protease and alkaline protease.

In yet a more particular embodiment, the invention provides a milk protein hydrolysate that comprises a peptide from 10 to 20 amino acids and comprising the amino acid sequence VLGPVRGPFP (SEQ ID NO: 1), and comprises a peptide of formula (I) X$^1$W, said protein hydrolysate is capable of inhibiting neprilysin enzyme with an IC50 from 0.005 to 0.600 mg/ml, and obtainable by a method comprising the steps of:

(a) adding to a composition comprising milk protein, said milk protein comprising milk alpha-casein and milk beta-casein in a total protein concentration from 3 % to 20 % weight/volume (w/v), at least a mixture of neutral protease and alkaline protease with a ratio of both enzymes from 5:1 to 1:5, the amount of neutral protease per mass unit of total protein in the milk protein composition from 2 x 10$^{-3}$ AU/g to 2 x 10$^{-1}$ AU/g and the amount of alkaline protease from 6 x 10$^{-3}$ AU/g to 6 x 10$^{-1}$ AU/g; and
(b) incubating at a constant pH from 6 to 8 and to a temperature from 45 ° C to 55 °C the mixture of (a) to hydrolysate the milk protein until a degree of hydrolysis (DH) of the composition is from 5.0 to 40.0 %; wherein the hydrolysate is capable of inhibiting neprilysin enzyme with an IC50 from 0.005 to 0.600 mg/ml.

In another particular embodiment of the aspect defined by a milk protein hydrolysate that comprises a peptide of formula (I), said hydrolysate is obtainable by a method comprising thermolysisn as protease system in step (a).

In a particular embodiment, the milk protein hydrolysate obtainable by the method of the invention comprises a peptide selected from the group consisting of a milk beta-casein peptide from 10 to 20 amino acids and comprising the amino acid sequence VLGPVRGPFP (SEQ ID NO: 1); a milk beta-casein peptide from 11 to 17 amino acids and comprising SEQ ID NO: 14; a milk alpha-casein peptide of formula (I) X$^1$W, wherein X$^1$ is selected from leucine (L) and isoleucine (I); and mixtures of said peptides.

In a particular embodiment of the milk protein hydrolysate of the invention, optionally in combination with any embodiment below or above, the peptide comprising the amino acid sequence VLGPVRGPFP (SEQ ID NO: 1) is selected from the group consisting of SEQ ID NO: 1, 2, 4-12. All these peptide sequences derive from the hydrolysis of cow milk beta-casein of SEQ ID NO: 3. SEQ ID NO: 2 corresponds to VLGPVRGPFPI. SEQ ID NO: 1 and 4-12 are those depicted in Table 2 of the Examples.

In a particular embodiment, optionally in combination with any of the embodiments above or below, the milk protein hydrolysate comprises a peptide consisting in SEQ ID NO: 1.

In another particular embodiment, optionally in combination with any of the embodiments above or below, the milk protein hydrolysate comprises a peptide of formula (I) X$^1$W, wherein X$^1$ is leucine (L).

Yet in another particular embodiment the milk protein hydrolysate comprises a mixture of the peptide of formula (I) X$^1$W, wherein X$^1$ is leucine (L) and the peptide of SEQ ID NO: 1.

In a more particular embodiment, optionally in combination with any of the embodiments above or below, the milk protein hydrolysate further comprises a peptide which consists in MHQPHQPLPPT (SEQ ID NO: 14). As above indicated this milk beta-casein peptide of SEQ ID NO: 14 is known for being active against rotavirus. This allows diarrhoea treatment caused by this microorganism. Milk protein hydrolysates, and particularly casein hydrolysates of the invention further comprising peptide of SEQ ID NO: 14 assure facing diarrhoea by means of inhibiting neprilysin and, at the same time, by focusing on any possible rotavirus infection. Thus, they focus the disease by to alternative mechanisms in case rotavirus infection is present.

As above exposed, it is also an aspect of the invention an edible composition comprising any of the milk protein hydrolysates as defined above. In a particular embodiment, the edible composition is a nutritional composition. In a more particular embodiment, the nutritional composition is selected from the group consisting of dietary supplements, oral rehydration serums, food additives, baby cereals and infant formulas.

In a preferred embodiment, the nutritional composition is an infant formula. Preferred compositions of infant formulas comprising the milk protein hydrolysates of the invention are depicted in the Examples below.

The milk protein hydrolysates of the invention are for use in the prevention and/or treatment of diarrhoea. In a particular embodiment, the milk protein hydrolysates are for use in the hypersecretory diarrhoea caused by increased cAMP concentration in intestinal cells that finally promotes secretion of salts and water into the intestine lumen. This secretion provokes the loss of faeces consistency. Agents such as enterotoxins, vasoactive intestinal peptide, or prostaglandin E2 trigger an increase in cyclic AMP levels, leading to hypersecretion of water and electrolytes. Enkephalins are released in an attempt to reduce the levels of cyclic AMP and thus counteract this hypersecretion. The enkephalins exert their effect via the delta opioid receptor, sited in several cells of the enteric nervous system (ENS). However, their inactivation by enkephalinase (neprylisin), sited mainly in enterocytes, limits the antisecretory activity of enkephalins. The enkephalinase inhibitor, racecadotril, prevents the breakdown of enkephalins and prolongs their antisecretory effect. Without being bound to theory, the inventors propose that the milk protein hydrolysates of the invention act in a similar way than racecadotril.

Thus, it can be said that the prevention and/or treatment of diarrhoea, in particular hypersecretory diarrhoea (also named intestinal hypersecretion) by means of the milk protein hydrolysates of the invention, is performed by inhibition of the enzyme neprilysin. Diarrhoea is faced in a different way than reducing intestine motility, which can be of interest in some type of population. Moreover, reduced intestine motility is usually the cause of co-lateral infections.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention.

EXAMPLES

EXAMPLE 1. Obtention of a milk protein hydrolysate of the invention and produced with neutral protease (Neutrase®) and alkaline protease (Alcalase®).

400 g of cow skimmed milk powder free of lactose (Innova Food Ingredients S.A.) were weighted in two bottles (2L each). Deionized water was added up to the final volume of 2 L while stirring (magnetic stirring) to avoid cake or lump formation. This gave rise to a 20 % weight/volume of skimmed milk in each bottled. Protein contents of skimmed milk was of 34 % (g / 100 g), which resulted in a percentage weight/volume (w/v) in the final solution for the hydrolysis of 6.8 %.

Resulting 4 L were added to a Biostat Bplus and heated to 50 °C while stirring at 400 rpm. pH control (pH= 7) with a solution of NaOH 5M was activated once 50 °C were reached. Then, stirring was lowered to 300 rpm until ending hydrolysis. 4 ml of each of the enzymes Alcalase® 2.4 L FG (Novozymes) and Neutrase® 0.8 L (Novozymes) were added, to a final concentration at initial of reaction of $1.47 \times 10^{-2}$ ml of enzyme /g protein in mixture, that corresponded, respectively to $1.47 \times 10^{-2}$ AU/g of Neutrase® 0.8 L , and $4.41 \times 10^{-2}$ AU/g of Alcalase® 2.4 L).

For the analysis of the hydrolysates aliquots (11 ml) were taken at DH= 0, 5, 8, 10, 15, 20, 25, 30, and 34 %. Aliquots were kept in a thermoblock at 100 °C for 15 minutes to inactivate proteases, homogenized and frozen at - 20 °C.

Inhibition of neprilysin was analysed for each of the aliquots as indicated below.

Same hydrolysate was scaled in a pilot assay. For this production, 100 L of skimmed milk powder free of lactose at 20 % (20 kg/100 L), and with a total protein contents of 34 g for 100 g of milk, were hydrolysed using 100 ml of Alcalase® 2.4 L FG (Novozymes) and 100 ml of Neutrase® 0.8 L (Novozymes) at 50 °C and maintaining pH at 7.0 (with NaOH 5 M and according to pH-stat method in Biostat Bplus). DH was monitored. At 30 minutes DH was of 21.09 % and 50 L (Sample N+2_Monells1) were removed in order to further deactivate proteases. Resting 50 L continued hydrolysis until 90 minutes, when a DH of 33.14 % was finally achieved, and at the end proteases were also deactivated (Sample N+2_Monells2). Both samples were dried.

Both hydrolysates (DH = 21.09 % and DH = 33.14 %) were analysed for inhibition of neprilysin.

Inhibition of neprylisin with the hydrolysate samples (the both of pilot assay production and those of aliquots with a DH= 5, 8, 10, 15, 20, 25, 30, and 34 % produced at laboratory) was analysed in vitro using a 96-well plate comprising recombinant human neprilysin (rhNeprilysin) and a fluorogenic substrate, the peptide Mca-RPPGFSAFK[Dnp] -OH (SEQ ID NO: 15), carrying the fluorescent label 7-Methoxycoumarin and the quencher dinitrophenyl lysine (R&D Systems, Inc., Minneapolis, MN 55413 USA) that emits fluorescence once hydrolysed. Due to sensitivity of the assay, each of the laboratory samples (stored at -20 °C) were heated at 96 °C for 20 minutes in order to assure that proteases (Alcalase® and Neutrase®) were inactive. This fluorogenic enzymatic assay with the fluorogenic peptide is performed at 37 °C, in a fluorescence plate reader (Infinite M200, Tecan) at 37°C with excitation at 320 nm and emission at 405 nm. When the enzyme is inhibited, quenched fluorogenic substrate it is not broken down and fluorescence signal is reduced. This fluorogenic technology is also known as FRET (Forster Resonance Energy Transfer).

Specific protocol and materials corresponded to: Assay Buffer (62.5 mM Tris, 0.0625% Brij-35, pH 9.0); Recombinant human Neprilysin (R&D Systems, Inc.); Substrate: Mca-RPPGFSAFK[Dnp]-OH (R&D Systems, Inc.) prepared at 2 mM stock in DMSO; F16 Black Maxisorp Plate (Nunc); Fluorescent Plate Reader Infinite M200 (Tecan). rhNeprilysin to 0.125 μg/ml was diluted in Assay Buffer. Fluorogenic peptide Substrate was diluted to 25 μM in Assay Buffer. The black well plate was loaded with 25 μl of each sample and 50 μl of 0.125 μg/ml rhNeprilysin. All samples are assayed in triplicate. Reaction was started after 10 minutes by adding 50 μl of 25 μM Substrate. As a Substrate Blank 50 μl of Substrate and 50 μl of Assay Buffer were loaded. When the plate reached 37 °C, readings were performed at excitation and emission wavelengths of 320 nm and 405 nm (top read). The data obtained after 40 minutes were used to calculate enzyme activity.

Accordingly, for each of the samples produced (at laboratory and pilot assay) several dilutions were prepared (with deionized water) for assaying the percentage of neprilysin inhibition. As usually, assayed dilutions were as follows to plot a percentage of inhibition curve: no dilution, 1/10, 1/30, 1/50, 1/100, 1/500 and 1/1000. Each of the dilutions were assayed in the enzymatic fluorogenic assay as indicated above.

Table 1 (see below) shows IC50 values determined from the curves of percentage (%) of neprilysin inhibition versus protein concentration (mg/ml) in the assayed sample (dilution) of the hydrolysates obtained with Alcalase® and Neutrase® and having different DH.

Table 1

| DH (%) | IC50 (mg/ml) |
|---|---|
| 0 (No hydrolysis) | Cannot be calculated (practically no inhibition) |
| 5 | 0.4015 // 0.3800 (the second from a frozen sample obtained before performance of assay in this Example 1, but with the same conditions) |
| 8 | 0.4083 |
| 10 | 0.3965 |
| 15 | 0.3716 |
| 20 | 0.3051 |
| 25 | 0.2652 |
| 30 | 0.2784 |
| 34 | 0.2723 |
| 21.09 % (Sample N+2_Monells1) (Pilot assay) | 0.0233 or 0.031 if heated to homogenize thermal treatment (see below) |
| 33.14 % (Sample N+2_Monells2) (Pilot assay) | 0.0216 |

In the laboratory series it can be seen that the greater the DH the lower the IC50 values. This means that a higher DH implies a higher inhibition degree until a DH =25 % is reached, when IC50 values remain asymptotically constant.

Differences between similar DH of the laboratory series and that of the pilot assay (Sample N+2_Monells1 and Sample N+2_Monells2) could be the result of the differential thermal treatment (pilot scale samples were not submitted at 96 °C prior to enzyme inhibition assay). For this reason, determination of the IC50 with the pilot assay sample having a DH = 21.09 % was repeated, but including the step of previously heating the sample at 96 °C. IC50 was of 0.031 mg/ml. Therefore, differences are not due to the previous thermal process. Anyway, this proves that at pilot study (previous step to industrial scale) and with the same parameters (enzymes, protein concentration, pH, and temperature) interesting results can be obtained with the hydrolysates in terms of enzyme inhibition, even with the same DH.

Noteworthy is the fact that a high difference in terms of IC50 values (inhibition) is observable between any hydrolysated sample and the non-hydrolysated sample (DH = 0 %), even at low DH values. Low DH values having already an inhibitory effect, minimize the risk of including in the milk protein hydrolysates of the invention a great percentage by weight of low molecular weight peptides that may confer a bitter taste.

EXAMPLE 2. Peptide composition of the hydrolysates obtained with neutral protease (Neutrase®) and alkaline protease (Alcalase®).

With the aim of resolving the active peptides causing neprilysin inhibition, analysis of the peptide composition of the hydrolysated was performed by isolating active fractions obtained from molecular exclusion chromatography. Besides, analysis of the peptide composition was also done by reverse phase high performance liquid chromatography (RP-HPLC).

Milk protein hydrolysates with DH = 21.09 % and 33.14 % were resuspended (10 % m/v) and filtered through a cut-off of 3 kDa (Vivaspin 2, Sartorius Stedim). Fractions with a molecular weight lower than 3 kDa (filtrate) were injected to a molecular size exclusion column (Superdex 30 pg, GE Healthcare) and fractions were collected. Column solvent was sodium phosphate buffer 50 mM, NaCl 0.15M. FIG. 1 and 2 show the chromatogram of the injected samples.

Fractions were previously analysed in an independent assay (not shown) for detecting neprilysin inhibition. It was determined that if further purified by RP-HPLC, greater inhibition extension was obtained (thus a lower IC50). Mass Spectrometry (MS) analysis revealed that the peptide LW was present in one of these fractions (F5).

LW dipeptide derives from hydrolysis of cow milk alpha-casein of SEQ ID NO: 13, which corresponds to the sequence in UniprotKB/Swiss-Prot database of accession number P02663, Version 114 of database of April 16, 2014, and Version 2 of July 1, 1989 for sequence version.

For this reason F5's of the hydrolysates with DH = 21.09 % and 33.14 % were also analysed by RP-HPLC (C-18 column, 50 $\mu$l of each sample previously concentrated from initial 12 ml to 1 ml). Solvent system included solvent A: water-TFA 1000:1, and solvent B (acetonitrile-TFA: 1000:1). Elution was performed starting with 100 % of A and reducing it using solvent B until a proportion of 30 % of B vs A. As a control, a solution with only peptide LW (from GenScript) was used at 0.04 mg/ml. The dipeptide elutes at t = 27.6 minutes. FIGs. 3 and 4 show how both control and F5 fractions completely superpose, thus proving that it is present in the hydrolysates.

It was also assayed the IC50 value of the LW peptide. Several dilutions were prepared (with deionized water) for assaying the percentage of neprilysin inhibition. A sample of 1 mg/ml LW and the following dilutions were analyzed: 1/2, 1/3, 1/5, 1/6, 1/7, 1/8, 1/10, 1/15, 1/20, 1/30, 1/50 and 1/100. IC50 is determined by FRET (Forster Resonance Energy Transfer) as explained in Example 1, using the fluorogenic peptide substrate (Mca-RPPGFSAFK[Dnp]-OH; R&D Systems, Inc.) to detect enzyme (recombinant human neprilysin, R&D Systems, Inc.) activity. The assay is done in the fluorescence plate reader (Infinite M200, Tecan) at 37°C with excitation at 320 nm and emission at 405 nm. IC50 was of $2.69 \times 10^{-2}$ mg/ml (84.76 $\mu$M).

Also as comparative example the IC50 of the active metabolite of racecadotril, DL-Thiorphan (Sigma-Aldrich Co. St. Louis, MO) was determined. IC50 was of $3.075 \times 10^{-5}$ mg/ml (0.1214 $\mu$M).

Although being DL-Thiorphan more active than LW or than any of the proposed milk protein hydrolysates, it is interesting to note that with the proposed hydrolysates or compositions comprising them, diarrhoea, in particular hypersecretory diarrhoea can also effectively be treated by means of neprilysin inhibition. This approach avoids secondary effects and drawbacks associated with Racecadotril, such as poor resuspension of granulates, poor aqueous solubility, dissolution rate limited absorption, and previously described side-effects such as headache, erythema multiform, tongue oedema, face oedema, lip oedema, urticaria, tonsillitis, papular rash, pruritus, prurigo, angioedema, eyelid oedema, erythema nodosum and toxic skin eruption.

As can be seen in FIG. 5, in which in addition it is compared the % of inhibition of several dilutions of the milk protein hydrolysate of Example 1 with a DH = 5 %, even the milk protein hydrolysate of DH = 5 % produced a higher neprilysin inhibition than the same dilution but of LW, both containing 0.04 mg/ml of the dipeptide. Thus, this makes evident that other than LW peptides in the hydrolysate inhibit the enzyme.

A HPLC-ESI-MS/MS with ion trapping analysis of laboratory and pilot assay hydrolysates of Example 1 revealed that all samples comprised, among others, the peptides of Table 2.

An HPLC analysis was done in an HPAgilent 1100 System (Agilent Technologies) equipped with a quaternary pump (Agilent Series 1100). As acquisition data system it was used the software ChemStation (Agilent Technologies). The used column was a reverse phase Hi-Pore C18 column (250 x 4,6 mm d.i., 5 $\mu$m of particle size) (Bio-Rad Laboratories, Richmond, CA, EEUU), and to increase sensitivity a low dimensioned column was used (150 mm x 2.1 mm Inertsil 5 ODS3 C18 with 5 $\mu$m of particle size (Varian, Bergen op Zoom, The Netherlands)). Solvent A was a mixture of water and trifluoroacetic acid (1000 : 0.37), and solvent B was a mixture of acetonitrile and trifluoroacetic acid (1000 : 0.27). HPLC equipment was coupled to a mass spectrometry detector type Esquire-3000 (Bruker Daltonik GmbH, Bremen, Alemania). The hydrolysates were eluted through the first column with a flow rate of 0.8 ml/min, with a lineal gradient of 0% to 45% of solvent B in A in 65 minutes. With the second column, the gradient was 0% to 45% of solvent B in A in 120 minutes. Solvent absorbance was monitored at a wavelength of 214 nm, and at the detector onset, the flow was passed to the mass spectrometry nebulizer (flow 0.2 ml/min). $N_2$ and helium were used as nebulizer and dryer gases in the mass spectrometer at a pressure of $5 \times 10^{-3}$ Pa and the mass spectra were acquired in a range from 100-1500 m/z. Capillary was maintained with a voltage of 4 kV. The signal of the analysis was the mean of 25 spectra, and for the MS(n) analysis a mean of 5 spectra was used, being n the number of generations of ions to be analysed. The intensity limit to perform MS(n) analysis was of 10000. Precursor ions were isolated in a range of 4 m/z, and they were fragmented with a voltage slope from 0.3 to 2.0 V. Spectral data were processed and transformed to mass values with the Data Analysis (version 3.0, Bruker Daltoniks) software. The software BioTools (version 2.1, Bruker Daltoniks) was used to process MS(n) spectra, and to perform the peptide sequencing.

The peptides listed in Table 2 are derived from the cow milk beta-casein of SEQ ID NO: 3 (*Bos Taurus*), which corresponds to the sequence in UniprotKB/Swiss-Prot database of accession number P02666, Version 126 of database release of April 16 2014, and Version 2 of the sequence of July 1 1989.

Table 2. Most represented peptides in cow milk casein hydrolysates obtainable by Neutrase® and Alcalase®

| SEQ ID NO: 1 | VLGPVRGPFP |
|---|---|
| SEQ ID NO: 4 | QEPVLGPVRGPFPI |
| SEQ ID NO: 5 | QEPVLGPVRGPFP |
| SEQ ID NO: 6 | YQEPVLGPVRGPFPIIV |
| SEQ ID NO: 7 | YQEPVLGPVRGPFPI |
| SEQ ID NO: 8 | YQEPVLGPVRGPFP |
| SEQ ID NO: 9 | LYQEPVLGPVRGPPIIV |
| SEQ ID NO: 10 | LYQEPVLGPVRGPFP |
| SEQ ID NO: 11 | LLYQEPVLGPVRGPFPIIV |
| SEQ ID NO: 12 | LLYQEPVLGPVRGPFP |

All these milk beta-casein peptides from 10 to 20 amino acids and comprising SEQ ID NO: 1, derived from C-terminal end of beta-casein (*Bos Taurus*), and they were hypothesized as having inhibitory effect on neprilysin activity.

Another peptide mainly represented in all the milk protein hydrolysates obtainable with the method using neutral protease and alkaline protease was that of SEQ ID NO: 14.

Inhibitory effect was assayed for peptide of SEQ ID NO: 1, as a sequence present in all the peptide sequences SEQ ID NO: 1-12 of Table 2. The assay was performed with the in vitro R&D Systems test previously disclosed in Example 1, and following the prescribed protocols of the test. Inhibitory effect was detected by the progressive extinction of fluorescence, as indicated in Example 1. Fluorescence values were determined at 40 minutes of reaction at 37 °C (recombinant human neprilysin with the fluorogenic substrate in the presence of several dilutions of the peptide of SEQ ID NO: 1). Controls included basal fluorescence derived from the peptide of SEQ ID NO: 1. Next table 3 shows the assayed concentrations of the peptide of SEQ ID NO: 1 and the percentage of inhibition of the enzyme (neprilysin). From these data it was calculated an IC50= 713 $\mu$M.

Table 3. Inhibitory effect of peptide of SEQ ID NO: 1

| Concentration peptide of SEQ ID NO: 1 (M) | % of inhibition |
|---|---|
| $1.26 \times 10^{-3}$ | 64.75 |
| $6.30 \times 10^{-4}$ | 46.62 |
| $3.15 \times 10^{-4}$ | 29.48 |
| $1.26 \times 10^{-4}$ | 13.22 |
| $9.00 \times 10^{-5}$ | 9.41 |
| $6.30 \times 10^{-5}$ | 7.37 |
| $1.26 \times 10^{-5}$ | 1.49 |

EXAMPLE 3.

Obtention of a milk protein hydrolysate comprising the active peptide VLGPVRGPFPI (SEQ ID NO: 2) produced with Thermolysin.

Another milk casein hydrolysate obtainable by the process of the invention was manufactured in a Lambda Minifor (Lambda Laboratory Instruments) with 1 L of commercial liquid lactose free milk (3 g of protein in 100 ml of milk).

Reaction conditions were fixed at 50 °C and pH 7.0 as in Example 1. In this case, 1 ml of Thermolysin (Protex 14L, Genencor) was used until a DH= 10% was reached (according to pH-stat method).

Peptide composition of the hydrolysate obtained with thermolysin was performed by HPLC-ESI-MS/MS with ion trapping as explained in Example 2. Peptides derived from both beta-casein and alpha-casein were identified.

From beta-casein, it was detected the peptide of SEQ ID NO: 2, including SEQ ID NO: 1, and also the peptide of SEQ ID NO: 14.

From alpha-casein, it was detected the dipeptide LW.

Inhibition of neprilysin was assayed as for the milk protein hydrolysates of Examples 1 and 2. The value obtained for IC50 was of 0.35422 mg/ml.

EXAMPLE 4. Comparative example. Inhibitory effect of a commercial casein hydrolysate

Casein hydrolysate known as CE90STL from DMV International (Delhi-NY) was also assayed for its neprilysin inhibitory activity.

CE90STL was resuspended to a concentration of 7.84 g/100 ml. Considering that the hydrolysates has 86.7% of protein and the 1/5 dilution needed for the neprilysin inhibition test, final protein concentration in the fluorogenic enzymatic assay was of 13.6 mg/ml. Dilutions 1/2, 1/5, 1/10, 1/20, 1/30, 1/50, 1/100, and no dilution (SD) were performed in order to test the percentage of neprilysin inhibition and further calculating the IC50 value of the hydrolysate. Calculated IC50 (following the same fluorogenic enzymatic assay as in the preceeding Examples) was of 1.595 mg/ml.

CE90STL has a DH of 39 %, according to the product data sheet, and it comprises 78 % by weight of peptides with a molecular weight lower than 500 Daltons (Da).

Thus, milk protein hydrolysates of the present invention have a higher inhibitory effect than other commercial milk hydrolysates, particularly commercial milk casein hydrolysates, and they represent good options for treating diarrhoea by means of neprylisin inhibition when forming part of edible compositions or as such.

EXAMPLE 5. Comparative example. Inhibitory effect of commercial milk protein hydrolysates

The following commercial products were tested in order to quantify the value of IC50 in an enzymatic assay against recombinant neprylisin:

- Lacprodan DI-3071 (Aria Foods®)- milk whey hydrolysate
- LE80GF whey, Hyvital Whey Easy-to-Digest 100 (DOMO®) - milk whey hydrolysate (DH= 15 %)

Powdered formulas were resuspended at a final concentration of 8.5 g/100 ml. Original samples have 80 % of protein. Considering the 1/5 dilution needed for the neprilysin inhibition test, final protein concentration in the fluorogenic enzymatic assay was of 13.6 mg/ml. Dilutions ½, 1/5, 1/10, 1/30, 1/50, 1/100, and no dilution (SD) were prepared for each sample and assayed as indicated in preceding examples, with the fluorogenic enzymatic assay.

Next table 4 shows IC50 values calculated from the dilutions assayed in the fluorogenic enzymatic assay. Comparison with two milk protein hydrolysates according to the invention is done:

Table 4.

| Sample | IC50 (mg/ml) |
|---|---|
| Lacprodan DI-3071 | 1.50 |
| LE80GF | 1.31 |
| Sample N+2_Monells1 (DH=21.09%) | 0.031 |
| Protein hydrolysate of Example 1 with DH=5 % | 0.380 |

As deducible from this Table 4, commercial hydrolysates behave an IC50 40-fold to 50-fold that of Sample N+2_Monells1 (DH=21.09%). Thus, although commercial hydrolysates have a residual neprilysin inhibitory effect, those of the present invention are much greater, even in case of a low DH, as deducible from the IC50 of the protein hydrolysate with DH= 5 %.

EXAMPLE 6. Infant formula with the protein milk hydrolysate of the invention.

Next Table 5 shows a detailed composition of an infant formula manufactured with the milk protein hydrolysate of the invention.

Table 5. Infant formula for treating diarrhoea:

| | UNITS | 100 g POWDER | 100 ml Dil.: 14,0% |
|---|---|---|---|
| TOTAL PROTEINS | g | 14,6 | 2,0 |

(continued)

|  | UNITS | 100 g POWDER | 100 ml Dil.: 14,0% |
|---|---|---|---|
| MILK PROTEIN HYDROL (DH=21.09%) | g(94,5%) | 13,8 | 1,9 |
| RICE PROTEINS | g(4,1 %) | 0,6 | 0,1 |
| FRUITS PROTEINS | g(1,4 %) | 0,2 | 0,03 |
| TOTAL FAT | g | 23,0 | 3,2 |
| LINOLEIC ACID | mg | 3128 | 438 |
| a-LINOLENIC ACID | mg | 253 | 35 |
| MCT | g | 6,1 | 0,9 |
| TOTAL CARBOHYDRATES | g | 53,0 | 7,4 |
| MALTODEXTRIN | g | 28,3 | 3,9 |
| GLUCOSE | g | 11,4 | 1,6 |
| SUGARS FROM FRUITS | g | 6,3 | 0,9 |
| PREGELATINIZED STARCH | g | 7,0 | 1,0 |
| FIBER | g | 3,3 | 0,5 |
| CHOLINE | mg | 100 | 14,0 |
| TAURINE | mg | 33 | 4,6 |
| INOSITOL | mg | 45 | 6,3 |
| L-CARNITINE | mg | 17 | 2,4 |

| **MINERALS** | UNITS | 100 g POWDER | 100 ml Dil.: 14,0% |
|---|---|---|---|
| TOTAL MINERALS | g | 3,5 | 0,5 |
| SODIUM | mg | 250 | 35 |
|  | mEq |  | 1,52 |
| POTASSIUM | mg | 700 | 98 |

|  | UNITS | 100 g POWDER | 100 ml |
|---|---|---|---|
|  | mEq |  | 2,51 |
| CHLORINE | mg | 380 | 53 |
|  | mEq |  | 1,5 |
| CALCIUM | mg | 490 | 69 |
| PHOSPHOROUS | mg | 270 | 38 |
| IRON | mg | 5,0 | 0,7 |
| MAGNESIUM | mg | 42 | 5,9 |
| ZINC | mg | 5,0 | 0,7 |
| COPPER | mcg | 320 | 45 |
| IODINE | mcg | 100 | 14,0 |
| MANGANESE | mcg | 100 | 14,0 |
| SELENIUM | mcg | 10,7 | 1,5 |
| FLUORIDE | mcg | 275 | 39 |
| RENAL SOLUTE LOAD RATIO Ca/P | mOsm/l | 1,8 | 135 |

| **VITAMINS** | UNITS | 100 g POWDER | 100 ml Dil.: 14,0% |
|---|---|---|---|
| VITAMIN A | mcg | 450 | 63 |
|  | IU | 1500 | 210 |
| VITAMIN D | mcg | 7,5 | 1,1 |
|  | IU | 300 | 44 |

(continued)

| VITAMINS | UNITS | 100 g POWDER | 100 ml Dil.: 14,0% |
|---|---|---|---|
| VITAMIN E | mg | 10,0 | 1,4 |
| | IU | 14,9 | 2,1 |
| VITAMIN K | mcg | 42 | 5,9 |
| VITAMIN B1 | mcg | 500 | 70 |
| VITAMIN B2 | mcg | 600 | 84 |
| VITAMIN B6 | mcg | 400 | 56 |
| VITAMIN B12 | mcg | 1,0 | 0,14 |
| VITAMIN C | mg | 70 | 9,8 |
| FOLIC ACID | mcg | 60 | 8,4 |
| PANTOTHENIC ACID | mg | 3,2 | 0,4 |
| NIACINE | mg | 5,0 | 0,7 |
| BIOTINE | mcg | 12,0 | 1,7 |
| D. E. PROT. | | 12,1% | |
| FAT | | 42,8% | |
| CARBOHYDRATES | | 43,8% | |
| FIBER | | 1,4% | |
| ENERGY | kcal | 484 | 67 |
| | kJ | 2027 | 282 |

EXAMPLE 7. Treatment of induced acute diarrhoea in rats with the hydrolysates of the invention

The effect of the hydrolysates of the invention was tested in a model of induced acute diarrhoea. For this test, female Wistar rats were used (8 weeks old). Animals were provided with 7 weeks by Harlan (Barcelona, Spain) and they rested in the Animal Experimental Service of Facultad de Farmacia de la Universidad de Barcelona (UB) under controlled temperature and humidity conditions with a light:dark cycle of 12 h:12 h with water and food ad libitum. The assay has been conducted under institutional guidelines for animal experimentation established by the Ethical Commitment of Animal Experimentation in the Universidad de Barcelona (CEEA-UB).

Previously to the assay, rats were weighed and randomly distributed to the cages. Haematocrit was also determined as basal value before inducing diarrhoea. Previously to the assay rats were starved for 16-24 hours. Different sub-serial of animals were assayed, each having n=28 for group (serie). Although the weight was reduced during the assay, the loss was detected in all groups, and it is hypothesized due to the starving period. No differences among groups were detected in the haematocrit.

The following experimental groups/series were tested:

Table 6. Experimental groups

| Group | p.o administration (-1h)./ animal Kg | Induced diarrhoea | Preparation of product |
|---|---|---|---|
| Healthy animals reference (RS) | Mineral water (vehicle) (10 ml/Kg) | --- | |
| Diarrheic animals reference (RD) | Mineral water (vehicle) (10 ml/Kg) | 1.5 ml castor oil | |
| Hydrolysate 1 (H1) (Laboratorios Ordesa, S.L.) | N+2_Monells1 (DH=21.09%). (8.5 g/Kg) | 1.5 ml castor oil | Hydrolysate previously weighed is pulverized in a mortar. Water is |
| | | | added to get 8.5 g/ml |
| Hydrolysate 2 (H2) (Laboratorios Ordesa, S.L.) | N+2_Monells2 (DH=33.14 %) (8.5 g/Kg) | 1.5 ml castor oil | Hydrolysate previously weighed is pulverized in a mortar. Water is added to get 8.5 g/ml |

(continued)

| Group | p.o administration (-1h)./ animal Kg | Induced diarrhoea | Preparation of product |
|---|---|---|---|
| Peptide (P) (LifeTein®) | LW (0.4 g/Kg) | 1.5 ml castor oil | Dissolved in mineral water at room temperature to the desired final concentration of 0.04 g/ml |
| Peptide plus (P+) (LifeTein®) | LW (0.8 g/Kg) | 1.5 ml castor oil | Dissolved in mineral water at room temperature to the desired final of 0.08 g/ml concentration |
| Non-hydrolysated (skimmed and lactose free)milk (L) (negative control) | Powdered bovine milk (6.5 g/Kg) | 1.5 ml castor oil | In mineral water to the desired final concentration of 0.65 g/ml |
| Racecadotril (R) (positive control) (Sigma Aldrich@) | 40 mg/Kg | 1.5 ml castor oil | Disoolved in Ethanol:Cremophor® EL: water (10:10:80) to get 4 mg/ml |

Before starting the assay it was checked that no diarrhoea was initially present.

Preventive treatment was administered peroral (p.o) 1 h before inducing diarrhoea using a gastric probe (the volume was given at a rate of 10 ml/Kg of animal)

Experimental induced acute diarrhoea was performed by administering 1.5 ml/animal of castor oil (Sigma-Aldrich@). The effect was assessed 1 h post-induction and systemically each hour for 8 hours. The following variables were determined: number of defecations, type (consistency, colour, form, presence or absence of mucus), punctuation (0-4 according to a diarrhoea index), presence or absence of diarrhoea. There were also determined the diarrhoea index (ID), diarrhoea incidence, the percentage of diarrhoeic defecations, as well as the severity of diarrhoeic process.

The diarrhea index (ID) was estimated as follows: ID= 0, healthy animals/dry defecation; ID=1, slightly wet defecation; ID=2-4 clearly diarrhoea with different grades of decomposition, from wet dark soft defecation (ID=2) to wet clear soft defecation with mucus (ID=3) and to wet decoloured soft defecation practically mucus as a whole (ID=4). In FIG. 7 ID is correlated with defecation appearance.

ID values $\geq$ 2 indicate diarrhoea
ID values < 2 indicate no diarrhoea

Another tested parameter was the diarrhoea incidence corresponding to the percentage of animals with diarrhoea (ID $\geq$ 2) in relation to total animals receiving castor oil.

None of the animals of healthy reference group (RS) presented diarrhoea symptoms. RD group 10 % of animals developed diarrhoea 1 hour after castor oil administration (post-induction, PI), and it increased along time (50 % at 2 hours PI, 70 % at 3 h PI, and getting a maximal of 90 % at 4 h PI). The tendency was maintained up to the end of the assay (24 h) and no improvement was observed.

Animals receiving the hydrolysates of the invention (H1 or H2) had a meaningful lower diarrhoea incidence in relation to RD at 2, 3, 4 and 5 h PI (p<0.05).

Pre-treatment with LW (P group) didn't reduce significantly diarrhoea in respect of RD group. Double dose (P+ group) reduced incidence being of 0 % for the first 4 h PI and low in relation to RD group at 5, 6 and 7 h PI (p<0.05).

Severity of diarrhoeic process corresponds to de ID expressed as Area under curve (AUC) for a specified time period after diarrhoea induction and for each group (G, X-axis). As can be seen in FIG. 8, wherein severity is illustrated in the 7 groups at several time ranges (0-3 h PI in FIG. 8 (A); and 0-8 h PI in FIG. 8 (B)), pre-treatment with any of H1 or H2 clearly diminished the severity of diarrhoeic process in any of the assayed time periods in relation with RD group (p<0.05). Pre-treatment with LW (0.8 g/Kg; P+ group) gave rise to the maximal effect between groups. As expected, group R (racecadotril group) presented a milder severity of diarrhoeic process than RD group, and being similar to that of group P+. Negative controls (L group) presented a severity of diarrhoeic process similar to that of RD group. Data were also collected for periods from 0-4 h PI, 0-5 h PI, 0-6 h PI and 0-24 h PI. Although data not shown, the same pattern of severity was observed at any period.

The percentage of diarrhoeic defecations (ID$\geq$2) was also determined for each group. The percentage was calculated with total defecations along a specified time period after diarrhoea induction.

Results are depicted in FIG. 9, wherein the percentage of diarrhoeic defecations in relation to the total of defecations (% ID$\geq$2 /T) for a period is illustrated for each group (G), during periods from 0 to 3 h PI (FIG. 9A) and from 0 to 8 h post induction (FIG. 9B).

The groups H1, H2 and R had a percentage of diarrhoeic defecations lower in both studied period.

Statistical analysis was performed by PASW®Statistics 18 software. Global comparative analysis between groups was performed with Kruskal-Wallis test and further with the non-parametric U test of Mann-Whitney. It was stablished as meaningful level a p value of p<0.05.

EXAMPLE 8. Intestine motility test

Intestine motility was evaluated in rats as in Example 7. The groups were coincident (n=7) except of the omission of R group and that only P+ (LW 0.8 g/Kg) was tested. The control group included rats treated with loperamide (Lop) at a dose of 2 mg/Kg, and rats treated with the vehicle (water) and with non-hydrolysed cow milk (L group). Loperamide is an anti-diarrhoeic drug that delays intestinal transit and it acts as a positive control in this experiment.

After 30 minutes of the administration of the products under study (P+, L, Lop, H1, H2, water) intestine transit was determined by administering 1 ml of a suspension at 1 % w/v of activated charcoal (Sigma-Aldrich®) in arabic gum (Acofar®, 5 %).

20 minutes later, animals were sacrificed by exsanguination and small intestine was extracted (from pilor to ileocecal). Small intestine was longitudinally disposed over a clean surface. Intestinal distance of charcoal was measured as well as the total intestine length. Results for each animal were expressed as distance of charcoal in relation to intestine length, in percentage form.

FIG. 10 shows in bars for each tested group (G) the distance of charcoal in small intestine, expressed as percentage of distance in relation to the total length of the intestine (D/L (%)).

From this FIG. 10 it is deduced that in RS group the distance was near $60.3 \pm 1.86$ % in relation to the small intestine length.

Previous administration of any of H1, H2 and P+, as well as of non-hydrolysed milk (L) does not modify intestinal transit in relation to RS group.

On the other hand and as predicted, loperamide administration (2 mg/Kg p.o. positive control) delayed intestinal transit in relation to RS group. In particular, with an intestinal transit of $37.8 \pm 3.49$ % (inhibition of 37 %, p<0.01 vs RS)

All these data allow confirming that the hydrolysates of the invention act in a diarrhoeic process not by inhibiting intestine motility but by other ways, in particular by inhibition of neprylisin. As above exposed, this has the advantage of facing diarrhoea with a safe mode, since proliferation of microorganism (i.e. some pathogen ones) due to a reduction in intestine motility and evacuation, is avoided.

Statistical analysis was performed by PASW®Statistics 18 software. Global comparative analysis between groups was performed with Kruskal-Wallis test and further with the non-parametric U test of Mann-Whitney. It was stablished as meaningful level a p value of p<0.05.

REFERENCES CITED IN THE APPLICATION

- US 5492899.
- EP2046149.
- Altschul, S. F., et. al. "Gapped BLAST and PSI-BLAST: a new generation of proteína database search programms", Nucleic Acids Research - 1997, Vol. No. 25, pp.: 3389 -3402
- Dhanda et al., "Functional characterization and specific effects of peptides on enzymatic activity of a DPP-III homologue from goat brain" Journal of Enzyme Inhibition and Medicinal Chemistry -2008, Vol. No. 23(2), pp.: 174-181.

**Claims**

1. A milk protein hydrolysate, for use in the prevention and/or treatment of diarrhoea, wherein the protein hydrolysate is capable of inhibiting neprilysin enzyme with an IC50 from 0.005 to 0.600 mg/ml, and it is obtainable by a method comprising the steps of:

(a) adding to a composition comprising milk protein that comprises milk casein selected from the group consisting of beta-casein, alpha-casein, and mixtures thereof, in a total milk protein concentration from 3 % to 20 % weight/volume (w/v), at least a protease system that is active at an optimal pH from 5.5 to 8.5, said protease system comprising a protease selected from the group consisting of thermolysin, or a mixture of neutral protease and alkaline protease; and

(b) incubating at a constant pH from 6 to 8 and to a temperature from 45 ° C to 55 °C the mixture of (a) to hydrolysate the milk protein until a degree of hydrolysis (DH) of the composition is from 1.0 to 40.0 %;

wherein DH is calculated by means of the following formula:

$$DH = B \times Nb \times (1/a) \times 1/MP \times 1/Htot \times 100;$$

wherein

B is the volume in millilitres (ml) of consumed base used for titrating released amino groups during hydrolysis of the composition comprising milk protein,

Nb is the normality of the base used for titrating,

1/a is the average degree of dissociation of the amino groups related with the pK of said amino groups at a particular pH and temperature, MP is the amount in grams of the composition comprising milk protein to be incubated with the protease system, and

Htot in milliequivalents per g (meq/g) is the sum of the millimoles of individual amino acids per gram of protein associated with the composition comprising milk protein; and

wherein the IC50 is the concentration of the hydrolysate that inhibits 50 % of the neprilysin enzyme activity, said IC50 determined by a fluorogenic enzymatic assay with a fluorescence excitation wavelength of 320 nm and with a fluorescence emission wavelength of 405 nm, the assay performed at 37 °C with dilutions of hydrolysate samples from no dilution to a dilution of at least 1/100 in water, in order to obtain a curve of percentage of inhibition of the enzyme per assayed hydrolysate dilution.

2. The milk protein hydrolysate for use according to claim 1, wherein:

(i) when the composition comprising milk casein of step (a) comprises milk beta-casein, then the milk casein hydrolysate after step (b) comprises a peptide from 10 to 20 amino acids and comprising the amino acid sequence VLGPVRGPFP (SEQ ID NO: 1);

(ii) when the composition comprising milk casein of step (a) comprises milk alpha-casein, then the milk casein hydrolysate after step (b) comprises a peptide of formula (I) $X^1W$, wherein $X^1$ is selected from leucine (L) and isoleucine (I); and

(iii) when the composition comprising milk casein of step (a) comprises a mixture of beta-casein and alpha-casein, then the milk casein hydrolysate after step (b) comprises a mixture of the peptides as disclosed in (i) and (ii).

3. The milk protein hydrolysate for use according to any of claims 1-2, wherein the composition comprising milk protein is cow milk.

4. The milk protein hydrolysate for use according to any of claims 1-3, wherein the protease system is a mixture of neutral protease and alkaline protease, and the amount of enzyme expressed as Anson Units (AU) of added neutral protease per mass unit in grams (g) of total protein in the milk protein composition is from $2 \times 10^{-3}$ AU/g to $2 \times 10^{-1}$ AU/g, and the amount of added alkaline protease per mass unit (in grams) of total protein in the milk protein composition is from $6 \times 10^{-3}$ AU/g to $6 \times 10^{-1}$ UA/g .

5. The milk protein hydrolysate for use according to any of claims 1-4, wherein the protease system is a mixture of neutral protease and alkaline protease and wherein the ratio of neutral protease : alkaline protease is from 5:1 to 1:5.

6. A milk protein hydrolysate capable of inhibiting neprilysin enzyme with an IC50 from 0.005 to 0.600 mg/ml, for use in the prevention and/or treatment of diarrhoea, wherein said milk protein hydrolysate is obtainable by a method comprising the steps of:

(a) adding to a composition comprising milk protein, said milk protein comprising milk beta-casein and a total protein concentration from 3 % to 20 % weight/volume (w/v), at least a mixture of neutral protease and alkaline protease with a ratio of both enzymes from 5:1 to 1:5, the amount of neutral protease per mass unit of total protein in the milk protein composition from $2 \times 10^{-3}$ AU/g to $2 \times 10^{-1}$ AU/g and the amount of alkaline protease from $6 \times 10^{-3}$ AU/g to $6 \times 10^{-1}$ AU/g; and

(b) incubating at a constant pH from 6 to 8 and to a temperature from 45 ° C to 55 °C the mixture of (a) to hydrolysate the milk protein until a degree of hydrolysis (DH) of the composition is from 5.0 to 40.0 %; wherein the milk protein hydrolysate after step (b) comprises the milk beta-casein peptide from 10 to 20 amino acids and comprising the amino acid sequence VLGPVRGPFP (SEQ ID NO: 1), and a milk beta-casein peptide from 11 to 17 amino acids and comprising the sequence MHQPHQPLPPT (SEQ ID NO: 14); wherein the IC50 and the DH are as defined in claim 1.

7. A milk protein hydrolysate that comprises a peptide from 10 to 20 amino acids and comprising the amino acid sequence VLGPVRGPFP (SEQ ID NO: 1), and comprises a peptide of formula (I) $X^1W$, wherein $X^1$ is selected from leucine (L) and isoleucine (I), for use in the prevention and/or treatment of diarrhoea, wherein said hydrolysate is obtainable by a method comprising the steps of:

(a) adding to a composition comprising milk alpha-casein and milk beta-casein in a total protein concentration from 3 % to 20 % weight/volume (w/v), at least a protease system that is active at an optimal pH from 5.5 to 8.5, said protease system comprising a protease selected from a mixture of neutral protease and alkaline protease, or thermolysin; and

(b) incubating at a constant pH from 6 to 8 and to a temperature from 45 ° C to 55 °C the mixture of (a) to hydrolysate the milk protein until a degree of hydrolysis (DH) of the composition is from 1.0 to 40.0 %;

wherein the hydrolysate is capable of inhibiting neprilysin enzyme with an IC50 from 0.005 to 0.600 mg/ml, the IC50 and the DH being as defined in claim 1.

8. An edible composition comprising the milk protein hydrolysate as defined in any of claims 6-7, for use in the prevention and/or treatment of diarrhoea.

9. The edible composition for use according to claim 8, which is a nutritional composition selected from the group consisting of a dietary supplement, an oral rehydration serum, a food additive, baby cereals and infant formula.

10. A pharmaceutical composition which comprises a therapeutically effective amount of the milk protein hydrolysate as defined in any of claims 6-7, together with appropriate amounts of pharmaceutical acceptable excipients and/or carriers, for use in the prevention and/or treatment of diarrhoea.

**Patentansprüche**

1. Ein Milchproteinhydrolysat zur Verwendung in der Prävention und/oder Behandlung von Durchfall, wobei das Proteinhydrolysat das Enzym Neprilysin bei einer IC50 von 0,005 bis 0,600 mg/ml inhibieren kann und mittels eines Verfahrens gewonnen werden kann, das folgende Schritte umfasst:

(a) hinzufügen von mindestens einem Proteasesystem, das bei einem optimalen pH-Wert von 5,5 bis 8,5 aktiv ist einer Zusammensetzung umfassend Milchprotein, welches Milchcasein umfasst, das ausgewählt ist aus der Gruppe bestehend aus beta-Casein, alpha-Casein, und Mischungen davon, bei einer gesamten Milchproteinkonzentration von 3 % bis 20 % Gewicht/Volumen (w/v), wobei das Proteasesystem eine Protease umfasst, die ausgewählt aus der Gruppe bestehend aus Thermolysin, oder einer Mischung aus neutraler Protease und alkalischer Protease ist; und

(b) inkubieren der Mischung von (a) bei einem konstanten pH-Wert von 6 bis 8 und bei einer Temperatur von 45 °C bis 55 °C, um das Milchprotein zu hydrolysieren, bis ein Hydrolysengrad (DH) der Zusammensetzung von 1,0 bis 40,0 % reicht;

wobei DH anhand der folgenden Formel berechnet wird:

$$DH = B \times Nb \times (1/a) \times 1/MP \times 1/Htot \times 100;$$

wobei

B das Volumen in Milliliter (ml) der zur Titration von freigesetzten Aminogruppen während der Hydrolyse der Zusammensetzung umfassend Milchprotein verbrauchten Base ist,

Nb die Normalität der zur Titration verwendeten Base ist,

1/a der mit dem pK-Wert der Aminogruppen bei einem bestimmten pH-Wert und einer bestimmten Temperatur verknüpfte durchschnittliche Dissoziationsgrad der Aminogruppen ist,

MP die Menge, in Gramm, der mit dem Proteasesystem zu inkubierenden Zusammensetzung umfassend Milchprotein ist, und Htot, in Milliäquivalenten pro g (meq/g), die Summe der Millimole von einzelnen Aminosäuren pro Gramm des mit der Zusammensetzung umfassend Milchprotein verknüpften Proteins ist; und

wobei die IC50 die Konzentration des Hydrolysats ist, welche 50 % der Aktivität des Enzyms Neprilysin inhibiert, wobei die IC50 mittels eines fluorogenen enzymatischen Testverfahrens mit einer Fluoreszenz-Anregungswellenlänge von 320 nm und mit einer Fluoreszenz-Emissionswellenlänge von 405 nm festgestellt wird, wobei der Test bei 37 °C mit Dilutionen der Hydrolysatproben durchgeführt wird, die von Null bis zu einer Dilution von mindestens 1/100 in Wasser reichen, um eine Kurve des Prozentsatzes der Inhibition des Enzyms pro untersuchter Hydrolysatsdilution zu erhalten.

2. Das Milchproteinhydrolysat zur Verwendung nach Anspruch 1, wobei:

(i) wenn die Zusammensetzung umfassend Milchcasein des Schritts (a) Milch-beta-Casein umfasst, das Hydrolysat des Milchcaseins nach dem Schritt (b) ein Peptid von 10 bis 20 Aminosäuren und umfassend die Aminosäuresequenz VLGPVRGPFP (SEQ ID NO: 1) umfasst;

(ii) wenn die Zusammensetzung umfassend Milchcasein des Schritts (a) Milch-alpha-Casein umfasst, das Hydrolysat des Milchcaseins nach dem Schritt (b) ein Peptid der Formel (I) $X^1W$ umfasst, wobei $X^1$ aus Leucin (L) und Isoleucin (I) ausgewählt ist; und

(iii) wenn die Zusammensetzung umfassend Milchcasein des Schritts (a) eine Mischung aus beta-Casein und alpha-Casein umfasst, das Hydrolysat von Milchcasein nach dem Schritt (b) eine Mischung der in (i) und (ii) offenbarten Peptide umfasst.

3. Das Milchproteinhydrolysat zur Verwendung nach einem der Ansprüche 1-2, wobei die Zusammensetzung umfassend Milchprotein Kuhmilch ist.

4. Das Milchproteinhydrolysat zur Verwendung nach einem der Ansprüche 1-3, wobei das Proteasesystem eine Mischung aus neutraler Protease und alkalischer Protease ist und die Menge an Enzym, ausgedrückt als Ansoneinheiten (AU) der hinzugefügten neutralen Protease pro Masseneinheit in Gramm (g) des gesamten Proteins in der Milchproteinzusammensetzung von $2 \times 10^{-3}$ AU/g bis $2 \times 10^{-1}$ AU/g reicht und wobei die Menge der hinzugefügten alkalischen Protease pro Masseneinheit (in Gramm) des gesamten Proteins in der Milchproteinzusammensetzung von $6 \times 10^{-3}$ AU/g bis $6 \times 10^{-1}$ UA/g reicht.

5. Das Milchproteinhydrolysat zur Verwendung nach einem der Ansprüche 1-4, wobei das Proteasesystem eine Mischung aus neutraler Protease und alkalischer Protease ist und wobei das Verhältnis neutrale Protease:alkalische Protease von 5:1 bis 1:5 reicht.

6. Ein Milchproteinhydrolysat, welches das Enzym Neprilysin bei einer IC50 von 0,005 bis 0,600 mg/ml hydrolysieren kann, zur Verwendung in der Prävention und/oder Behandlung von Durchfall, wobei das Milchproteinhydrolysat mittels eines Verfahrens gewonnen werden kann, das folgende Schritte umfasst:

(a) hinzufügen von mindestens einer Mischung aus neutraler Protease und alkalischer Protease bei einem Verhältnis der beiden Enzyme von 5:1 bis 1:5 einer Zusammensetzung umfassend Milchprotein, wobei das Milchprotein Milch-beta-Casein und eine gesamte Proteinkonzentration von 3 % bis 20 % Gewicht/Volumen (w/v) umfasst und wobei die Menge der neutralen Protease pro Masseneinheit des gesamten Proteins in der Milchproteinzusammensetzung von $2 \times 10^{-3}$ AU/g bis $2 \times 10^{-1}$ AU/g reicht und die Menge an alkalischer Protease von $6 \times 10^{-3}$ AU/g bis $6 \times 10^{-1}$ AU/g reicht; und

(b) inkubieren der Mischung von (a) bei einem konstanten pH-Wert von 6 bis 8 und bei einer Temperatur von 45 °C bis 55 °C, um das Milchprotein zu hydrolysieren, bis ein Hydrolysengrad (DH) der Zusammensetzung von 5,0 bis 40,0 % reicht; wobei das Milchproteinhydrolysat nach dem Schritt (b) das Milch-beta-Caseinpeptid von 10 bis 20 Aminosäuren und umfassend die Aminosäuresequenz VLGPVRGPFP (SEQ ID NO: 1) umfasst, und ein Milch-beta-Caseinpeptid von 11 bis 17 Aminosäuren und umfassend die Sequenz MHQPHQPLPPT (SEQ ID NO: 14) umfasst, wobei die IC50 und der DH wie im Anspruch 1 definiert sind.

7. Ein Milchproteinhydrolysat, welches ein Peptid von 10 bis 20 Aminosäuren und umfassend die Aminosäuresequenz VLGPVRGPFP (SEQ ID NO: 1) umfasst und ein Peptid der Formel (I) $X^1W$ umfasst, wobei $X^1$ aus Leucin (L) und Isoleucin (I) ausgewählt ist, zur Verwendung in der Prävention und/oder Behandlung von Durchfall, wobei das Hydrolysat mittels eines Verfahrens gewonnen werden kann, welches folgende Schritte umfasst:

(a) hinzufügen von mindestens einem Proteasesystem, das bei einem optimalen pH-Wert von 5,5 bis 8,5 aktiv ist einer Zusammensetzung umfassend Milch-alpha-Casein und Milch-beta-Casein bei einer gesamten Proteinkonzentration von 3 % bis 20 % Gewicht/Volumen (w/v), wobei das Proteasesystem eine Protease umfasst, die aus einer Mischung aus neutraler Protease und alkalischer Protease, oder Thermolysin ausgewählt ist; und
(b) inkubieren der Mischung von (a) bei einem konstanten pH-Wert von 6 bis 8 und bei einer Temperatur von 45 °C bis 55 °C, um das Milchprotein zu hydrolysieren, bis ein Hydrolysengrad (DH) der Zusammensetzung von 1,0 bis 40,0 % reicht;

wobei das Hydrolysat das Enzym Neprilysin bei einer IC50 von 0,005 bis 0,600 mg/ml inhibieren kann, wobei die IC50 und der DH wie im Anspruch 1 definiert sind.

8. Eine essbare Zusammensetzung umfassend das Milchproteinhydrolysat wie in einem der Ansprüche 6-7 definiert, zur Verwendung in der Prävention und/oder Behandlung von Durchfall.

9. Die essbare Zusammensetzung zur Verwendung nach Anspruch 8, welche eine Nährstoffzusammensetzung ausgewählt aus der Gruppe bestehend aus einem Nahrungsergänzungsmittel, einem oralen Rehydratationsserum, einem Nahrungsmittelzusatzstoff, Babygetreiden und Säuglingsanfangsnahrung ist.

10. Eine pharmazeutische Zusammensetzung, welche eine therapeutisch wirksame Menge des Milchproteinhydrolysats wie in einem der Ansprüche 6-7 definiert zusammen mit geeigneten Mengen an pharmazeutisch akzeptablen Hilfsstoffen und/oder Trägersubstanzen umfasst zur Verwendung in der Prävention und/oder Behandlung von Durchfall.

**Revendications**

1. Un hydrolysat de protéine du lait pour l'utilisation dans la prévention et/ou le traitement de la diarrhée, dans lequel l'hydrolysat de protéine est capable d'inhiber l'enzyme néprilysine avec une IC50 allant de 0,005 à 0,600 mg/ml et qui peut être obtenu moyennant un procédé comprenant les étapes de :

(a) ajouter à une composition comprenant de la protéine du lait qui comprend de la caséine du lait choisie dans le groupe constitué de la béta-caséine, l'alpha-caséine, et des mélanges de celles-ci, à une concentration de protéine du lait totale de 3 % à 20 % poids/volume (w/v), au moins un système de protéases qui est actif à un pH optimal de 5,5 à 8,5, ledit système de protéases comprenant une protéase choisie dans le groupe constitué de la thermolysine ou un mélange de protéase neutre et protéase alcaline ; et
(b) incuber à un pH constant de 6 à 8 et à une température de 45 °C à 55 °C le mélange de (a) pour hydrolyser la protéine du lait jusqu'à ce qu'un degré d'hydrolyse (DH) de la composition soit de 1,0 à 40,0 % ;

dans lequel DH est calculé moyennant la formule suivante :

$$DH = B \times Nb \times (1/a) \times 1/MP \times 1/Htot \times 100 ;$$

où

B est le volume en millilitres (ml) de base consommée utilisé pour titrer les groupes amino libérés pendant l'hydrolyse de la composition comprenant de la protéine du lait,
Nb est la normalité de la base utilisée pour le titrage,
1/a est le degré de dissociation moyen des groupes amino lié au pK desdits groupes amino à un pH donné et à une température donnée, MP est la quantité en grammes de la composition comprenant de la protéine du lait à être incubée avec le système de protéases, et

Htot en milliéquivalents par g (meq/g) est la somme des millimoles d'acides aminés individuels par gramme de protéine liée à la composition comprenant de la protéine du lait ; et

dans lequel la IC50 est la concentration de l'hydrolysat qui inhibe 50 % de l'activité de l'enzyme néprilysine, ladite IC50 étant déterminée moyennant un test enzymatique fluorogène avec une longueur d'onde d'excitation de fluorescence de 320 nm et avec une longueur d'onde d'émission de fluorescence de 405 nm, le test étant effectué à 37 °C avec des dilutions d'échantillons d'hydrolysat de dilution zéro à une dilution d'au moins 1/100 dans l'eau, afin d'obtenir une courbe de pourcentage d'inhibition de l'enzyme par dilution d'hydrolysat analysée.

2. L'hydrolysat de protéine du lait pour l'utilisation selon la revendication 1, dans lequel :

(i) lorsque la composition comprenant de la caséine du lait de l'étape (a) comprend de la béta-caséine du lait, alors l'hydrolysat de caséine du lait après l'étape (b) comprend un peptide de 10 à 20 acides aminés et comprenant la séquence d'acides aminés VLGPVRGPFP (SEQ ID NO: 1) ;
(ii) lorsque la composition comprenant de la caséine du lait de l'étape (a) comprend de la alpha-caséine du lait, alors l'hydrolysat de caséine du lait après l'étape (b) comprend un peptide de formule (I) $X^1W$, dans laquelle $X^1$ est choisi parmi la leucine (L) et l'isoleucine (I) ; et
(iii) lorsque la composition comprenant de la caséine du lait de l'étape (a) comprend un mélange de béta-caséine et alpha-caséine, alors l'hydrolysat de caséine du lait après l'étape (b) comprend un mélange des peptides tels que divulgués dans (i) et (ii).

3. L'hydrolysat de protéine du lait pour l'utilisation selon l'une quelconque des revendications 1-2, dans lequel la composition comprenant de la protéine du lait est du lait de vache.

4. L'hydrolysat de protéine du lait pour l'utilisation selon l'une quelconque des revendications 1-3, dans lequel le système de protéases est un mélange de protéase neutre et protéase alcaline, et la quantité d'enzyme exprimée en unités Anson (AU) de protéase neutre ajoutée par unité de masse en grammes (g) de protéine totale dans la composition de protéine du lait est de $2 \times 10^{-3}$ AU/g à $2 \times 10^{-1}$ AU/g, et la quantité de protéase alcaline ajoutée par unité de masse (en grammes) de protéine totale dans la composition de protéine du lait est de $6 \times 10^{-3}$ AU/g à $6 \times 10^{-1}$ UA/g.

5. L'hydrolysat de protéine du lait pour l'utilisation selon l'une quelconque des revendications 1-4, dans lequel le système de protéases est un mélange de protéase neutre et de protéase alcaline et dans lequel le rapport protéase neutre : protéase alcaline est de 5 : 1 à 1 : 5.

6. Un hydrolysat de protéine du lait capable d'inhiber l'enzyme néprilysine avec une IC50 allant de 0,005 à 0,600 mg/ml, pour l'utilisation dans la prévention et/ou le traitement de la diarrhée, dans lequel ledit hydrolysat de protéine du lait peut être obtenu moyennant un procédé comprenant les étapes de :

(a) ajouter à une composition comprenant de la protéine du lait, ladite protéine du lait comprenant de la béta-caséine du lait et une concentration de protéine totale de 3 % à 20 % poids/volume (w/v), au moins un mélange de protéase neutre et protéase alcaline avec un rapport de toutes deux enzymes allant de 5 : 1 à 1 : 5, la quantité de protéase neutre par unité de masse de protéine totale dans la composition de protéine du lait allant de $2 \times 10^{-3}$ AU/g à $2 \times 10^{-1}$ AU/g et la quantité de protéase alcaline allant de $6 \times 10^{-3}$ AU/g à $6 \times 10^{-1}$ AU/g; et
(b) incuber à un pH constant allant de 6 à 8 et à une température de 45 °C à 55 °C le mélange de (a) pour hydrolyser la protéine du lait jusqu'à ce qu'un degré d'hydrolyse (DH) de la composition soit de 5,0 à 40,0 % ; dans lequel l'hydrolysat de protéine du lait après l'étape (b) comprend le peptide de béta-caséine du lait ayant de 10 à 20 acides aminés et comprenant la séquence d'acides aminés VLGPVRGPFP (SEQ ID NO : 1), et un peptide de béta-caséine du lait ayant de 11 à 17 acides aminés et comprenant la séquence MHQPHQPLPPT (SEQ ID NO : 14), dans lequel la IC50 et le DH sont tels que définis dans la revendication 1.

7. Un hydrolysat de protéine du lait qui comprend un peptide ayant de 10 à 20 acides aminés et comprenant la séquence d'acides aminés VLGPVRGPFP (SEQ ID NO : 1), et qui comprend un peptide de formule (I) $X^1W$, dans laquelle $X^1$ est choisi parmi la leucine (L) et l'isoleucine (I), pour l'utilisation dans la prévention et/ou le traitement de la diarrhée, dans lequel ledit hydrolysat peut être obtenu moyennant un procédé comprenant les étapes de:

(a) ajouter à une composition comprenant de l'alpha-caséine du lait et de la béta-caséine du lait à une concentration de protéine totale de 3 % à 20 % poids/volume (w/v), au moins un système de protéases qui est actif à un pH optimal de 5,5 à 8,5, ledit système de protéases comprenant une protéase choisie parmi un mélange de protéase neutre et protéase alcaline, ou de la thermolysine ; et

(b) incuber à un pH constant de 6 à 8 et à une température de 45 °C à 55 °C le mélange de (a) pour hydrolyser la protéine du lait jusqu'à ce qu'un degré d'hydrolyse (DH) de la composition soit de 1,0 à 40,0 % ;

dans lequel l'hydrolysat est capable d'inhiber l'enzyme néprilysine avec une IC50 de 0,005 à 0,600 mg/ml, la IC50 et le DH étant tels que définis dans la revendication 1.

8. Une composition comestible comprenant l'hydrolysat de protéine du lait tel que défini dans l'une quelconque des revendications 6-7, pour l'utilisation dans la prévention et/ou le traitement de la diarrhée.

9. La composition comestible pour l'utilisation selon la revendication 8, qui est une composition nutritionnelle choisie dans le groupe constitué d'un supplément nutritionnel, un sérum de réhydratation oral, un additif alimentaire, des céréales pour bébés et un substitut du lait maternel.

10. Une composition pharmaceutique qui comprend une quantité thérapeutiquement efficace de l'hydrolysat de protéine du lait tel que défini dans l'une quelconque des revendications 6-7, conjointement avec des quantités appropriées d'excipients et/ou de véhicules pharmaceutiquement acceptables, pour l'utilisation dans la prévention et/ou le traitement de la diarrhée.

**FIG. 1**

**FIG. 2**

**FIG. 3**

**FIG. 4**

**FIG. 5**

**FIG. 6**

**FIG. 7**

A

B

FIG. 8

**FIG. 9**

(G)

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5492899 A
- EP 2046149 A
- WO 2009000899 A
- EP 14191179 A

### Non-patent literature cited in the description

- **TESCHEMACHER H. et al.** *Biopoly,* 1997, vol. 43, 99-117
- **NAGPAL R et al.** *Food Funct.,* 2011, vol. 2, 18-27
- **ALTSCHUL, S. F.** Gapped BLAST and PSI-BLAST: a new generation of proteína database search programms. *Nucleic Acids Research,* 1997, vol. 25, 3389-3402
- **DHANDA et al.** Functional characterization and specific effects of peptides on enzymatic activity of a DPP-III homologue from goat brain. *Journal of Enzyme Inhibition and Medicinal Chemistry,* 2008, vol. 23 (2), 174-181
- **ALTSCHUL, S. F.** Gapped BLAST and PSI-BLAST: a new generation of proteína database search programms. *Nucleic Acids Research,* 1997, vol. 25, 3389-3402